(19) Europäisches Patentamt
European Patent Office
Office européen des brevets

(11) EP 1 582 211 A1

(12) **EUROPEAN PATENT APPLICATION**
published in accordance with Art. 158(3) EPC

(43) Date of publication:
**05.10.2005 Bulletin 2005/40**

(21) Application number: **03780677.5**

(22) Date of filing: **03.12.2003**

(51) Int Cl.[7]: **A61K 31/416**, A61K 31/4439, A61K 31/444, A61K 31/497, A61P 3/10, A61P 9/00, A61P 9/10, A61P 21/00, A61P 25/00, A61P 25/14, A61P 25/16, A61P 25/28, A61P 43/00, C07D 231/56, C07D 401/06, C07D 401/14, C07D 409/14, C07D 403/12

(86) International application number:
**PCT/JP2003/015481**

(87) International publication number:
**WO 2004/050088 (17.06.2004 Gazette 2004/25)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **03.12.2002 JP 2002351345**

(71) Applicant: **KYOWA HAKKO KOGYO CO., LTD.**
**Tokyo 100-8185 (JP)**

(72) Inventors:
- **KANAI, Fumihiko, c/o Head Office**
  **Chiyoda-ku, Tokyo 100-8185 (JP)**
- **KUMAZAWA, Toshiaki, c/o Tokyo Research Lab.**
  **Machida-shi, Tokyo 194-8533 (JP)**
- **SAITO, Jun-ichi**
  **4051 Basel (CH)**
- **SHIMADA, Junichi,**
  **Pharmaceutical Research Inst.**
  **Nagaizumi-cho, Sunto-gun, Shizuoka 411 (JP)**
- **HIROSE, Ryo,**
  **Pharmaceutical Research Institute**
  **Nagaizu-cho, Sunto-gun, Shizuoka 411-8 (JP)**
- **ICHIMURA, Michio,**
  **c/o Tokyo Research Laboratories**
  **Machida-shi, Tokyo 194-8533 (JP)**

(74) Representative: **Casalonga, Axel et al**
**Bureau D.A. Casalonga - Josse,**
**Paul-Heyse-Strasse 33**
**80336 München (DE)**

(54) **JNK INHIBITOR**

(57) The present invention provides a JNK inhibitor comprising, as an active ingredient, an indazole derivative represented by Formula (I)

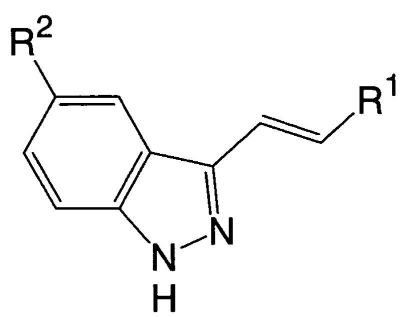

(I)

[wherein $R^1$ represents substituted or unsubstituted aryl or the like and $R^2$ represents a hydrogen atom, $NR^3R^4$ (wherein $R^3$ and $R^4$ may be the same or different and each represents a hydrogen atom, substituted or unsubstituted lower alkanoyl or the like), carboxy, lower alkenyl or the like] or a pharmaceutically acceptable salt thereof.

EP 1 582 211 A1

## Description

Technical Field

The present invention relates to JNK inhibitors which are useful for treatment of a brain neurodegenerative disorder or the like. The present invention also relates to indazole derivatives or pharmaceutically acceptable salts thereof which have JNK inhibitory activity and are useful for treatment of a brain neurodegenerative disorder or the like.

Background Art

JNK (c-Jun N-terminal kinase) is an enzyme which is activated by physical or chemical stresses such as hypertonic stimulation, ultraviolet rays or a protein synthesis inhibitor, or cytokine such as TNF-$\alpha$ (tumor necrosis factor-$\alpha$) or IL-1 (interleukin-1) and which phosphorylates Jun which is an AP-1 (activator protein-1) transcription factor to increase its transcription activity; and it is related to stress response and apoptosis. In particular, it is known that JNK plays an essential role in apoptosis of PC12 cells caused by removal of serum and in apoptosis mediated by ceramide and that JNK is activated during apoptosis of various cell strains and primary culture of striatum nerve cells of newborn rats *[Nippon Rinsho*, volume 56, page 1779 (1998); *Journal of Biological Chemistry*, volume 273, page 3756 (1998)].

It has been known at present that there are three subtypes of JNK: JNK1, JNK2 and JNK3, and it has also been reported that cell death of hippocampus by excitatory neurotoxin is significantly decreased in JNK3 knockout mice *[Nature*, volume 389, page 865 (1997)]. Therefore, JNK inhibitors have been thought to be useful as agents for treatment of a brain neurodegenerative disorder.

As JNK inhibitors, oxyindole derivatives, etc. have been known (WO 00/35906, WO 00/35909, WO 00/35921, etc.)

As indazole derivatives, various compounds have been known [Japanese Published Unexamined Patent Application (Kokai) No. 32059/1990, WO 01/53268, WO 02/10137, etc.] Among them, the compounds disclosed in WO 02/10137 are shown to have JNK inhibitory activity.

In Japanese Published Unexamined Patent Application (Kokai) No. 32059/1990, compounds represented by Formula (III)

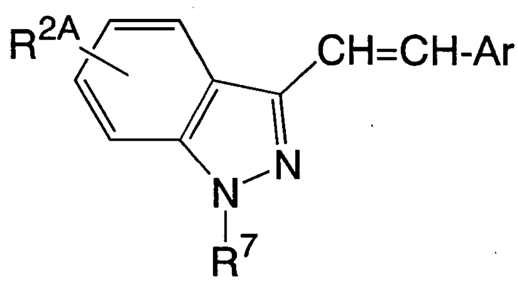

(III)

{wherein $R^{2A}$ represents a hydrogen atom, nitro, $NR^{3D}R^{4D}$ [wherein $R^{3D}$ and $R^{4D}$ may be the same or different and each represents a hydrogen atom, substituted or unsubstituted lower alkyl, or lower alkanoyl (the carbon number in the lower alkanoyl is 1 to 6) or the like] or the like, $R^7$ represents a hydrogen atom or the like, and Ar represents pyridyl, substituted or unsubstituted 2-oxochromenyl (the 2-oxochromenyl is bonded to an ethenyl group (-CH=CH-) on its benzene ring and substituent(s) on the 2-oxochromenyl group is lower alkyl having 1 to 6 carbon(s) or lower alkoxy having 1 to 6 carbons(s)), phenyl or substituted phenyl [substituents $Q^{1a}$, $Q^{2a}$ and $Q^{3a}$ in the substituted phenyl may be the same or different and each represents a hydrogen atom, halogen, lower alkyl having 1 to 6 carbon(s), hydroxy, lower alkoxy having 1 to 6 carbon(s), nitro, nitroso, carboxy, lower alkoxycarbonyl having 1 to 6 carbon(s), $NR^{3E}R^{4E}$ (wherein $R^{3E}$ and $R^{4E}$ have the same meanings as $R^{3D}$ and $R^{4D}$ def ined above, respectively) or $O(CH_2)_{nd}NR^{3D1}R^{4D1}$ (wherein nd is an integer of 1 to 6 and $R^{3D1}$ and $R^{4D1}$ have the same meanings as $R^{3D}$ and $R^{4D}$ above defined, respectively) or any two from the groups $Q^{1a}$ to $Q^{3a}$ are combined together to form $-O(CR^{3F}R^{4F})O-$ (wherein two terminal oxygen atoms are bonded to the phenyl group and are positioned ortho each other and $R^{3F}$ and $R^{4F}$ may be the same or different and each represents a hydrogen atom or lower alkyl having 1 to 6 carbon (s) or $R^{3F}$ and $R^{4F}$ are combined together to form an alkylene group having 4 to 5 carbons) ; with the proviso that $Q^{1a}$, $Q^{2a}$ and $Q^{3a}$ which are the substituents in the substituted phenyl are not simultaneously hydrogen atoms]} are disclosed.

In WO 01/53268, compounds having suppressive activity on cell differentiation represented by Formula (IV)

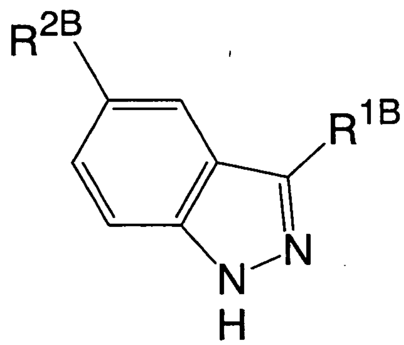

(IV)

[wherein $R^{1B}$ represents CH=CH-$R^{1C}$ (wherein $R^{1C}$ represents substituted or unsubstituted alkyl, substituted or unsubstituted aryl, substituted or unsubstituted heterocyclic group or the like) and $R^{2B}$ represents CH=CH-$R^{1C1}$ (wherein $R^{1C1}$ represents substituted or unsubstituted aryl, substituted or unsubstituted heteroaryl or the like)] are disclosed.

In WO 02/10137, compounds having JNK inhibitory activity represented by Formula (V)

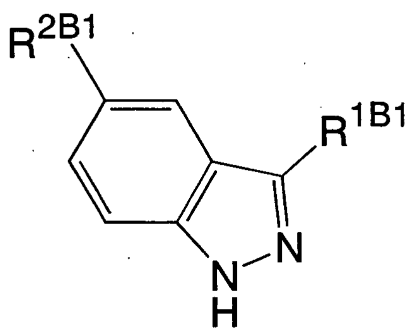

(V)

[wherein $R^{1B1}$ represents CH=CH-$R^{1D}$ (wherein $R^{1D}$ represents optionally substituted aryl, optionally substituted heteroaryl or the like) and $R^{2B1}$ represents a hydrogen atom, amino or the like] are disclosed.

**Disclosure of the Invention**

An object of the present invention is to provide JNK inhibitors which are useful for treatment of a brain neurodegenerative disorder or the like, and to provide novel indazole derivatives or pharmaceutically acceptable salts thereof which have JNK inhibitory activity and are useful for treatment of a brain neurodegenerative disorder or the like.

The present invention relates to the following (1) to (22).

(1) A JNK (c-Jun N-terminal kinase) inhibitor comprising, as an active ingredient, an indazole derivative represented by Formula (I)

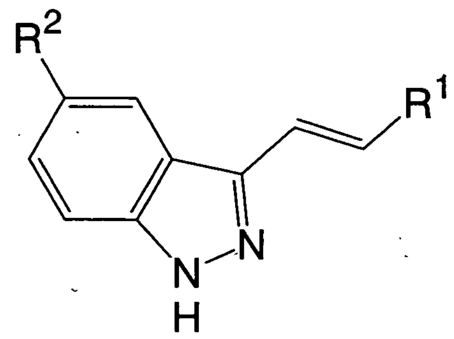

(I)

[wherein $R^1$ represents substituted or unsubstituted aryl or a substituted or unsubstituted heterocyclic group, and

$R^2$ represents

a) a hydrogen atom,
b) $NR^3R^4$ (wherein $R^3$ and $R^4$ may be the same or different and each represents a hydrogen atom, substituted or unsubstituted lower alkanoyl, substituted or unsubstituted aroyl, heteroaroyl, or lower alkoxycarbonyl),
c) carboxy,
d) $CONR^{3A}R^{3B}$ (wherein $R^{3A}$ and $R^{4B}$ may be the same or different and each represents a hydrogen atom, substituted or unsubstituted lower alkanoyl, substituted or unsubstituted aryl, substituted or unsubstituted aroyl, heteroaroyl or lower alkoxycarbonyl),
e) substituted or unsubstituted lower alkenyl or
f) substituted or unsubstituted lower alkynyl] or a pharmaceutically acceptable salt thereof.

(2) An indazole derivative represented by Formula (II)

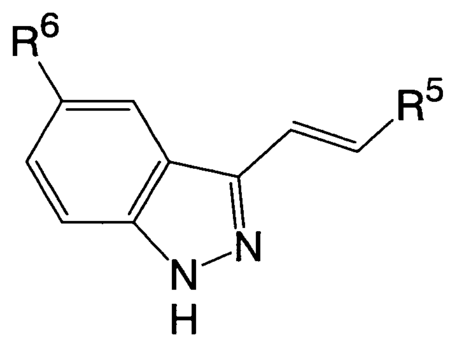

(II)

[wherein $R^5$ represents substituted or unsubstituted pyridyl, or aroylamino-substituted phenyl, and
$R^6$ represents

a) $NR^{3B}R^{4B}$ (wherein $R^{3B}$ and $R^{4B}$ may be the same or different and each represents a hydrogen atom, substituted or unsubstituted alkanoyl, substituted or unsubstituted aroyl or heteroaroyl),
b) carboxy,
c) $CONR^{3C}R^{3C}$ (wherein $R^{3C}$ and $R^{4C}$ may be the same or different and each represents a hydrogen atom or substituted or unsubstituted aryl),
d) substituted or unsubstituted lower alkenyl or
e) substituted or unsubstituted lower alkynyl] or a pharmaceutically acceptable salt thereof.

(3) A therapeutic agent for a brain neurodegenerative disorder comprising at least one indazole derivative or pharmaceutically acceptable salt thereof described in (1).

(4) The therapeutic agent for a brain neurodegenerative disorder according to (3), wherein the brain neurodegenerative disorder is the disease selected from cerebral infarction, Parkinson's disease, Alzheimer's disease, progressive supranuclear paralysis, AIDS encephalopathy, transmissible spongiform encephalopathy, multiple sclerosis, amyotrophic lateral sclerosis, multiple system atrophy, attention-deficit/hyperactivity disorder, Huntington's disease, a diabetic neurosis and a traumatic neurodegenerative disorder.

(5) A therapeutic agent for acute cerebral infarction comprising at least one indazole derivative or pharmaceutically acceptable salt thereof described in (1).

(6) A therapeutic agent for a brain neurodegenerative disorder comprising at least one indazole derivative or pharmaceutically acceptable salt thereof described in (2).

(7) The therapeutic agent for a brain neurodegenerative disorder according to (6), wherein the brain neurodegenerative disorder is the disease selected from cerebral infarction, Parkinson's disease, Alzheimer's disease, progressive supranuclear paralysis, AIDS encephalopathy, transmissible spongiform encephalopathy, multiple sclerosis, amyotrophic lateral sclerosis, multiple system atrophy, attention-deficit/hyperactivity disorder, Huntington's disease, a diabetic neurosis and a traumatic neurodegenerative disorder.

(8) A therapeutic agent for acute cerebral infarction comprising at least one indazole derivative or pharmaceutically acceptable salt thereof described in (2).

(9) A pharmaceutical composition comprising at least one indazole derivative or pharmaceutcally acceptable salt thereof described in (2).

(10) A JNK inhibitor comprising the indazole derivative or the pharmaceutically acceptable salt thereof described in (2).

(11) A method for treatment and/or prevention of a disease derived from activation of JNK, comprising a step of administering an effective amount of the indazole derivative or the pharmacologically acceptable salt thereof described in (1).

(12) A method for treatment and/or prevention of a brain neurodegenerative disorder, comprising a step of administering an effective amount of the indazole derivative or the pharmaceutically acceptable salt thereof described in (1).

(13) A method for treatment and/or prevention of acute cerebral infarction, comprising a step of administering an effective amount of the indazole derivative or the pharmaceutically acceptable salt thereof described in (1).

(14) A method for treatment and/or prevention of a disease derived from activation of JNK, comprising a step of administering an effective amount of the indazole derivative or the pharmaceutically acceptable salt thereof described in (2).

(15) A method for treatment and/or prevention of a brain neurodegenerative disorder, comprising a step of administering an effective amount of the indazole derivative or the pharmaceutically acceptable salt thereof described in (2).

(16) A method for treatment and/or prevention of acute cerebral infarction, comprising a step of administering an effective amount of the indazole derivative or the pharmaceutically acceptable salt thereof described in (2).

(17) Use of the indazole derivative or the pharmaceutically acceptable salt thereof described in (1) for the manufacture of a JNK inhibitor.

(18) Use of the indazole derivative or the pharmaceutically acceptable salt thereof described in (1) for the manufacture of an agent for treatment and/or prevention of a brain neurodegenerative disorder.

(19) Use of the indazole derivative or the pharmaceutically acceptable salt thereof described in (1) for the manufacture of an agent for treatment and/or prevention of acute cerebral infarction.

(20) Use of the indazole derivative or the pharmaceutically acceptable salt thereof described in (2) for the manufacture of a JNK inhibitor.

(21) Use of the indazole derivative or the pharmaceutically acceptable salt thereof described in (2) for the manufacture of an agent for treatment and/or prevention of a brain neurodegenerative disorder.

(22) Use of the indazole derivative or the pharmaceutically acceptable salt thereof described in (2) for the manufacture of an agent for treatment and/or prevention of acute cerebral infarction.

Hereinafter, the compounds represented by Formula (I) will be referred to as Compound (I). This will be similarly applied to the compounds of other formula numbers.

In the definition for each group of the compounds in the present specification, the alkyl moiety in the lower alkoxycarbonyl represents, for example, linear or branched alkyl having 1 to 8 carbon(s) such as methyl, ethyl, propyl, isopropyl, butyl, isobutyl, sec-butyl, tert-butyl, pentyl, neopentyl, hexyl, heptyl, or octyl.

The lower alkenyl represents, for example, linear or branched alkenyl having 2 to 8 carbons such as vinyl, allyl, butenyl, isobutenyl, pentenyl, hexenyl, heptenyl, or octenyl.

The lower alkynyl represents, for example, alkynyl having 2 to 8 carbons such as ethynyl, propynyl, butynyl,

pentynyl, hexynyl, heptynyl, or octynyl.

The lower alkanoyl represents, for example, linear or branched alkanoyl having 1 to 8 carbon (s) such as formyl, acetyl, propionyl, butyryl, isobutyryl, valeryl, isovaleryl, pivaloyl, hexanoyl, heptanoyl, or octanoyl.

The aryl moiety of the aryl, the aroyl and the aroylamino represents, for example, phenyl, naphthyl, or the like.

The heterocyclic group represents, for example, an aliphatic heterocyclic group such as pyrrolidinyl, piperidinyl, 1,2-dihydropyridyl, N-substituted or unsubstituted piperazinyl (the substituent on the nitrogen atom is lower alkyl, lower alkanoyl or the like, and the lower alkyl and the lower alkanoyl have the same meanings as defined above, respectively), morpholinyl, thiomorpholinyl, pyrazolinyl, oxazolinyl, dioxolanyl, tetrahydropyranyl or N-substituted or unsubstituted homopiperazinyl (the substituent on the nitrogen atom is lower alkyl, lower alkanoyl or the like, and the lower alkyl and the lower alkanoyl have the same meanings as defined above, respectively) or an aromatic heterocyclic group such as furyl, thienyl, pyrrolyl, pyridyl, imidazolyl, pyrazolyl, triazolyl, thiazolyl, oxazolyl, oxadiazolyl, pyrimidinyl, indolyl, cumarinyl, benzimidazolyl, benzoxazolyl, benzimidazolyl, benzoxazolyl, benzothiazolyl, quinolyl, isoquinolyl, quinazolinyl or pyrazinyl.

The heteroaryl moiety in the heteroaroyl represents an aromatic heterocyclic group such as furyl, thienyl, pyrrolyl, pyridyl, imidazolyl, pyrazolyl, triazolyl, thiazolyl, oxazolyl, oxadiazolyl, pyrimidinyl, indolyl, cumarinyl, benzimidazolyl, benzoxazolyl, benzothiazolyl, quinolyl, isoquinolyl, quinazolinyl or pyrazinyl.

Examples of the substituent (s) in the substituted lower alkenyl, the substituted lower alkynyl and the substituted lower alkanoyl, which may be the same or different and which is 1 to 3 in number, include

a) hydroxy,
b) lower alkoxy,
c) oxo,
d) carboxy,
e) lower alkoxycarbonyl,
f) $NR^8R^9$ [wherein $R^8$ and $R^9$ may be the same or different and each represents a hydrogen atom or lower alkyl, or $R^8$ and $R^9$ are combined together with the adjacent nitrogen atom to form a heterocyclic group (the heterocyclic group may contain an oxygen atom, a sulfur atom or another nitrogen atom)],
g) $CONR^{8A}R^{9A}$ (wherein $R^{8A}$ and $R^{9A}$ have the same meanings as $R^8$ and $R^9$ defined above, respectively),
h) substituted aryl [the substituent(s) in the substituted aryl is/are carboxy or lower alkoxycarbonyl, etc.],
i) a substituted or unsubstituted heterocyclic group [the substituent (s) in the substituted aryl is/are lower alkoxy etc.],
j) heteroaroyl,
k) arylsulfonyl, etc.

In the definition for the substituents in the substituted lower alkenyl, the substituted lower alkynyl and the substituted lower alkanoyl, the lower alkoxycarbonyl has the same meaning as defined above and the lower alkyl and the lower alkyl moiety in the lower alkoxy have the same meaning as the alkyl moiety of the lower alkoxycarbonyl defined above. The aryl, the heteroaryl and the heterocyclic group have the same meanings as defined above, respectively. Examples of the heterocyclic group formed together with the adjacent nitrogen atom (the heterocyclic group may contain an oxygen atom, a sulfur atom or another nitrogen atom) include, for example, pyrrolidinyl, morpholino, thiomorpholino, pyrazolidinyl, piperidino, piperazinyl, N-substituted piperazinyl [the substituent(s) on the nitrogen atom is/are lower alkyl or lower alkanoyl, etc.], homopiperazinyl, N-substituted homopiperazinyl [the substituent(s) on the nitrogen atom is / are lower alkyl or lower alkanoyl, etc.], pyrrolyl, indolyl and isoindolyl. The aryl moiety in the arylsulfonyl has the same meaning as the aryl defined above.

Examples of the substituent (s) in the substituted aryl, the substituted aroyl, the substituted pyridyl and the substituted heterocyclic group, which may be the same or different and which is 1 to 3 in number, include

a) substituted or unsubstituted lower alkoxy,
b) carboxy,
c) lower alkoxycarbonyl,
d) $NR^{10}R^{11}$ (wherein $R^{10}$ and $R^{11}$ may be the same or different and each represents a hydrogen atom, substituted or unsubstituted lower alkyl, aralkyl, lower alkanoyl, aroyl or lower alkoxycarbonyl),
e) $CONR^{10A}R^{11}A$ (wherein $R^{10A}$ and $R^{11A}$ have the same meanings as $R^{10}$ and $R^{11}$ defined above, respectively),
f) substituted or unsubstituted lower alkyl,
g) oxo,
h) formyl, etc.

In the definition for the substituents in the substituted aryl, the substituted aroyl, the substituted pyridyl and

the substituted heterocyclic group, the lower alkyl, the lower alkoxy, the lower alkoxycarbonyl, the lower alkanoyl and the aroyl have the same meanings as defined above, respectively. The aryl moiety in the aralkyl has the same meaning as the aryl defined above and the alkylene moiety in the aralkyl represents a group formed by removing one hydrogen atom from the lower alkyl defined above. Substituent (s) in the substituted lower alkyl and the substituted lower alkoxy have the same meaning as the substituent (s) in the substituted lower alkenyl defined above.

Examples of the pharmaceutically acceptable salts of Compound (I) include, for example pharmaceutically acceptable acid addition salts, metal salts, ammonium salts, organic amine addition salts, amino acid addition salts, etc. Examples of the acid addition salts are inorganic acid salts such as hydrochlorides, sulfates and phosphates and organic acid salts such as acetates, maleates, fumarates, tartrates, citrates, lactates, aspartates and glutamates. Examples of the metal salts are alkaline metal salts such as sodium salts and potassium salts, alkaline earth metal salts such as magnesium salts and calcium salts, aluminum salts and zinc salts; examples of the ammonium salts are ammonium salts and tetramethylammonium salts; examples of the organic amine addition salts are addition salts with morpholine and piperizine; and examples of the amino acid addition salts are addition salts with lysine, glycine and phenylalanine.

The process for producing Compound (II) will be illustrated bellow.

In the following reaction steps, structural formulae, tables, etc. , Me, Ac, Ph and Bz stand for methyl, acetyl, phenyl and benzoyl, respectively. Further, definitions for each groups in the following reaction steps have the same meanings as each group defined above, respectively, unless otherwise noted.

Furthermore, when the defined group changes under the conditions of the conducted process or is not suitable for conducting the reaction process in the following production methods, it is possible to obtain the targeted compound using a method for introduction and elimination of protective group commonly used in synthetic organic chemistry, etc. [e.g., Protective Groups in Organic Synthesis, by T. W. Greene, John Wiley & Sons, Inc. (1981)]. If necessary, the order of reaction steps such as introduction of substituent may be changed.

Compound (II) can be produced according to the following reaction steps.

**Production Method 1**

Compound (II) can be produced by the following process from Compound (A) obtained by a similar manner to the known method [e.g., *Journal of Organic Chemistry,* volume 52, page 19 (1987); and *Canadian Journal of Chemistry,* volume 51, page 792 (1973)].

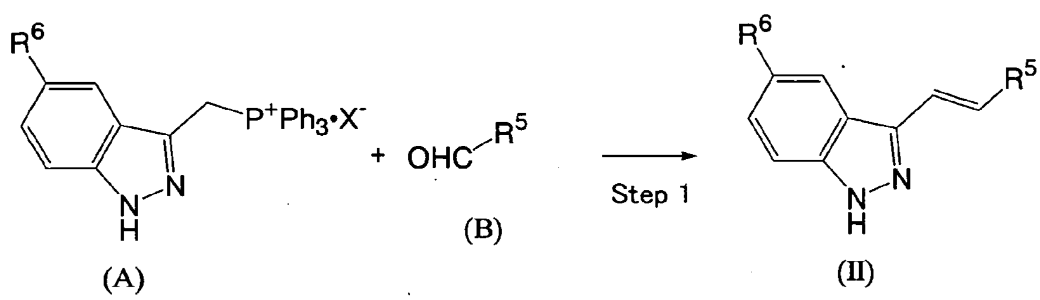


(wherein X represents chlorine, bromine or iodine and $R^5$ and $R^6$ have the same meaning as defined above, respectively)

Step 1

Compound (II) can be obtained by reacting Compound (A) with Compound (B) in a solvent such as methanol, ethanol, tetrahydrofuran or N,N-dimethylformamide or a mixture of these solvents in the presence of a base.

Potassium carbonate, potassium tert-butoxide, sodium hydride, etc. may be used as the base. To Compound (A), 1 to 10 equivalent(s) of Compound (B) and the base are used, respectively. The reaction is usually performed at a temperature between 0 and 100 °C and finishes in 1 to 72 hour(s).

**Production Method 2**

Among the Compound (II), Compound (IIb) having a specific functional group as $R^5$ or $R^6$ may be also pro-

duced by the following process from Compound (C) having other functional groups as $R^5$ or $R^6$ obtained by the production method 1 or another known method (such as Japan Published Unexamined Patent Application (Kokai) No. 32059/1990).

Although all compounds mentioned as Compound (IIb), etc. in the following Steps 2-1 to 2-8 are not always included in the scope of Compound (II), they are here called Compound (IIb) for the sake of convenience. Further, even among compounds called here Compound (C) in the following steps 2-1 to 2-8, there are compounds included in Compound (II).

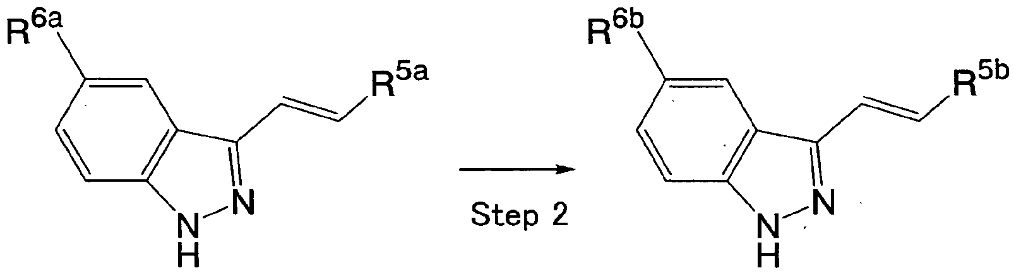

(C) (IIb)

(wherein $R^{5a}$, $R^{6a}$, $R^{5b}$ and $R^{6b}$ are the group defined in the following steps 2-1 to 2-8, respectively. When there is no particular definition therefor in the following steps 2-1 to 2 - 8 , $R^{5a}$ and $R^{5b}$ have the same meanings as $R^5$ and $R^{6a}$ and $R^{6b}$ have the same meanings as $R^6$, respectively)

Step 2-1

(In the step 2-1, at least one of $R^{5a}$ and $R^{6a}$ represents a substituent including lower alkoxycarbonyl and at least one of $R^{5b}$ and $R^{6b}$ represents a substituent including carboxy)

Compound (IIb) is obtained by subjectiing Compound (C) to hydrolysis in water or in a mixed solvent of water and methanol, ethanol, tetrahydrofuran or the like in the presence of a base such as sodium hydroxide or an acid such as hydrochloric acid.

To Compound (C), 0.1 to 10 equivalent (s) of the acid or the base is used. The reaction is usually performed at a temperature between 20 °C and 100 °C and finishes in 1 to 24 hour(s).

Step 2-2

(In the step 2-2, $R^{6a}$ represents nitro and $R^{6b}$ represents amino)

Compound (IIb) can be obtained by treating Compound (C) with a reducing agent such as tin or iron in a solvent such as water or ethanol or a mixture thereof or without solvent and in the presence of an acid such as concentrated hydrochloric acid or acetic acid, or subjecting Compound (C) to a reduction in a solvent such as water, methanol, ethanol, tetrahydrofuran or N, N-dimethylformamide or a mixture thereof , in the presence of a catalyst such as palladium-carbon, platinum dioxide or Raney nickel and in a hydrogen atmosphere or in the presence of hydrogen donor such as hydrazine hydrate and ammonium formate.

To Compound (C), 1 to 20 equivalent (s) of a reducing agent such as tin or iron, 0.5 to 100% by weight of the catalyst and 1 to 100 equivalent(s) of the hydrogen donor are used. The reaction is usually performed at a temperature between 0 °C and 100 °C and finishes in 1 to 72 hour(s).

Step 2-3

[In the step 2-3, at least one of $R^{5a}$ and $R^{6a}$ represents a substituent including carboxy and at least one of $R^{5b}$ and $R^{6b}$ represents a substituent including $CONR^{3D}R^{4D}$ (wherein $R^{3D}$ and $R^{4D}$ have the same meanings as $R^{3C}$ and $R^{4C}$ defined above, respectively)]

Compound (IIb) can be obtained by reacting Compound (C) with Compound (III) represented by $HNR^{3D}R^{4D}$ (wherein $R^{3D}$ and $R^{4D}$ have the same meanings as defined above, respectively) in a solvent such as dichloromethane, tetrahydrofuran, 1,4-dioxane, N,N-dimethylformamide, N-methylpiperidone or a mixed solvent thereof and in the presence of a condensing agent such as dicyclohexylcarbodiimide, 1-ethyl-3-(3-dimethylaminopropyl)carbodiimide hydrochloride, polymer-bound 1-ethyl-3-(3-dimethylaminopropyl) carbodiimide or triphenylphosphine oxide trifluoromethanesulfonic acid anhydride and an activator such as 1-hydrodxybenzotriazole or N-hydroxysuccinimide.

To Compound (C), 1 to 20 equivalent(s) of the condensing agent, the activator and Compound (III) are used, respectively. The reaction is usually perfomed at a temperature between -20 °C and 80 °C and finishes in 30 minutes to 24 hours. Incidentally, depending upon the type of Compound (III), it is also possible that Compound (III) is mixed with an activator to prepare a salt in advance, and then used in the reaction.

Step 2-4

[In the step 2-4, $R^{6a}$ represents amino and $R^{6b}$ represents a substituent including $NHCOR^{12}$ (wherein $R^{12}$ represents substituted or unsubstituted lower alkyl, substituted or unsubstituted aryl or heteroaryl)]

In the definition of $R^{12}$, lower alkyl, aryl and heteroaryl have the same meanings as defined above, respectively. The substituent in the substituted lower alkyl has the same meaning as the substituent in the substituted lower alkenyl defined above and the substituent in the substituted aryl has the same meaning as defined above.

Compound (IIb) can be obtained by reacting Compound (C) with Compound (IV) represented by $R^{12}COCl$ (wherein $R^{12}$ has the same meaning as defined above) or Compound (V) represented by $(R^{12}CO)_2O$ (wherein $R^{12}$ has the same meaning as defined above) in the presence of a base such as triethylamine, pyridine, p-dimethylaminopyridine, polyvinylpyridine, 4-morpholinomethylpolystyrene or 4-piperidinopolystyrene, or reacting Compound (C) with Compound (VI) represented by $R^{12}CO_2H$ (wherein $R^{12}$ has the same meaning as defined above) in the presence of a condensing agent such as dicyclohexylcarbodiimide, 1-ethyl-3-(3-dimethylaminopropyl)carbodiimide hydrochloride or polymer bound 1-ethyl-3-(3-dimethylaminopropyl)carbodiimide, and an activator such as 1- hydroxybenzotriazole or N-hydroxysuccinimide, in a solvent such as dichloromethane, tetrahydrofuran, 1,4-dioxane, N,N-dimethylformamide, N-methylpiperidone or mixture thereof.

To Compound (C), 1 to 20 equivalent(s) of the base, Compound (IV) or (V), the condensing agent, the activator and Compound (VI) are used, respectively. The reaction is usually performed at a temperature between -20°C and 80 °C and finishes in 30 minutes to 24 hours.

Step 2-5

(In the step 2-5, $R^{6a}$ represents halogen and $R^{6b}$ represents carboxy)

Compound (IIb) can be obtained by treating Compound (C) with a strong base such as sodium hydride or n-butyl lithium in a solvent such as tetrahydrofuran and then reacting with gaseous or solid carbon dioxide.

To Compound (C), 1 to 10 equivalent (s) of the strong base is used and 1 to 200 equivalent(s) of carbon dioxide is used. The reaction is usually performed at a temperature between -80°C and 30°C and finishes in 1 to 24 hour(s).

Step 2-6

(In the step 2-6 , $R^{6a}$ represents halogen and $R^{6b}$ represents substituted or unsubstituted lower alkynyl)

Compound (IIb) can be obtained by reacting Compound (C) with substituted or unsubstituted alkyne (the substituent in the substituted alkyne has the same meaning as the substituent in the substituted alkynyl defined above) in a solvent such as tetrahydrofuran, 1,4-dioxane, N,N-dimethylformamide, water, diethylamine or a mixture thereof, in the presence or absence of a palladium catalyst such as palladium acetate, bis(benzonitrile)dichloropalladium, bis (acetonitrile)dichloropalladium, dichlorobis(triphenylphosphine)palladium or tetrakis(triphenylphosphine)palladium, in the presence or absence of a ligand such as triphenylphosphine, tributylphosphine, tri(o-tolyl)phosphine or tri(tert-butyl)phosphine, in the presence or absence of a catalyst such as cuprous halide (halogen in the cuprous halide has the same meaning as defined above) and in the presence or absence of a base such as triethylamine, diethylamine or diisopropylamine.

To Compound (C), 1 to 10 equivalent(s) of substituted or unsubstituted alkyne, 0.01 to 10 equivalent(s) of the palladium catalyst and the ligand, and 0 to 10 equivalent(s) of cuprous halide and the base are used, respectively. The reaction is usually performed at a temperature between 0 °C and 150 °C and finishes in 1 to 120 hour(s).

Step 2-7

(In the step 2-7, at least one of $R^{5a}$ and $R^{6a}$ represents a substituent including formyl or lower alkoxycarbonyl and at least one of $R^{5b}$ and $R^{6b}$ represents a substituent including hydroxymethyl)

Compound (IIb) can be obtained by treating Compound (C) with a reducing agent such as sodium borohydride, lithium borohydride, lithium aluminum hydride or diisobutylaluminum hydride in a solvent such as water, methanol,

ethanol, 2-propanol, tetrahydrofuran, ether, dichloromethane or a mixture thereof.

To Compound (C), 1 to 10 eguivalent(s) of the reducing agent is used. The reaction is usually performed at a temperature between -78 °C and 100 °C and finishes in 5 minutes to 24 hours.

Step 2-8

(In the step 2-8, $R^{6a}$ is formyl and $R^{6b}$ is substituted or unsubstituted lower alkenyl)

Compound (IIb) can be obtained by reacting Compound (C) with Compound (VII) represented by $R^{13}CH_2P^+Ph_3 \cdot X^-$ (wherein X has the same meaning as defined above and $R^{13}$ represents a. substituent in the substituted lower alkenyl or a hydrogen atom), Compound (VIII) represented by $R^{13}CH^2P(O)(OR^{14})_3$ (wherein $R^{13}$ has the same meaning as defined above and $R^{14}$ is methyl or ethyl) or Compound (IX) represented by $R^{13}CH{=}PPh_3$ (wherein a $R^{13}$ has the same meaning as defined above) in a solvent such as methanol, ethanol, tetrahydrofuran, N,N-dimethylformamide, toluene or a mixture thereof in the presence or absence of a base.

Potassium carbonate, potassium tert-butoxide, sodium hydride, n-butyl lithium or the like may be used as the base. To Compound (C), 1 to 10 equivalent(s) of Compound (VII), Compound (VIII) or Compound (IX) is used and 1 to 10 equivalent (s) of the base is used, respectively. The reaction is usually performed at a temperature between 0°C and 100 °C and finishes in 1 to 72 hour(s).

Transformation of a functional group contained in $R^5$ or $R^6$ in Compound (II) and in the starting compound can also be conducted by other known methods [such as "Comprehensive Organic Transformations" by R. C. Larock, (1989)] in addition to the above-mentioned steps.

Compound (C) used as a starting material for the step 2-2 can be produced by the following step 3 (In the following formulae, Compound (C) which is used as a material for the step 2-2 is called Compound (E) for the sake of convenience)

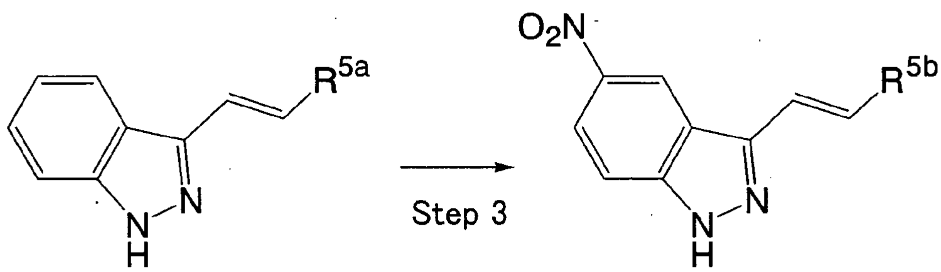


(D) (E)

Step 3

Compound (E) can be obtained by treating Compound (D) with a mixture of concentrated nitric acid and concentrated sulfuric acid.

To Compound (D), 5 to 100 equivalent concentrated nitric acid and concentrated sulfuric acid are used, respectively. The reaction is usually performed at a temperature between -10 °C and 20 °C and finishes in 1 to 24 hour(s).

Compound (II) having a desired functional group at a predetermined position can be obtained by conducting the above-mentioned methods in an appropriate combination thereof.

Compound (I) can be obtained by synthesizing in the similar manner to the above-mentioned production methods for Compound (II) or the known method or by purchasing commercially available products.

Isolation and purification of the products in the above-mentioned production methods can be conducted by an appropriate combination of usual methods used in organic synthesis such as filtration, extraction, washing, drying, concentrating, crystallization and various chromatographic techniques. Intermediates can be used in the following reaction step without further purification.

In Compound (I) and Compound (II), isomers such as position isomer, geometric isomer, tautomer and optical isomer may be present and any possible isomer and a mixture of such isomers in any ratio are used in the present invention or included in the present invention.

When it is desired to obtain a salt of Compound (I) or Compound (II), in the case where it is obtained in a form of a salt, this may be purified as it is, where it is obtained in a free form, it is dissolved or suspended in an appropriate solvent followed by adding an acid or a base thereto to form a salt.

Compound (I) or a pharmaceutically acceptable salt thereof and Compound (II) or a pharmaceutically acceptable salt thereof may be present in a form of an adduct with water or with various kinds of solvents, and such an adduct is also used or included in the present invention.

Specific examples of Compound (I) include Compound 1 and Compound 2. Specific examples of Compound (II) include the compounds of Examples other than the Compounds 1 and 2.

Table 1

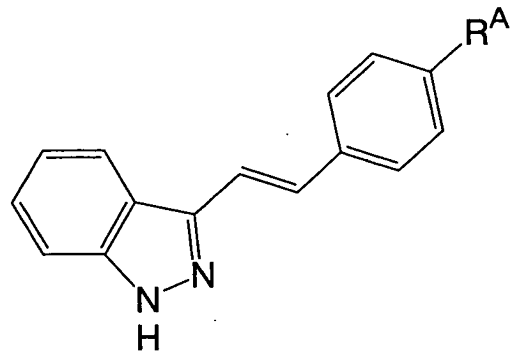

| Compound No. | $R^A$ | Salt |
|---|---|---|
| 1 | $OCH_2CH_2NMe_2$ | HCl |

Table 2

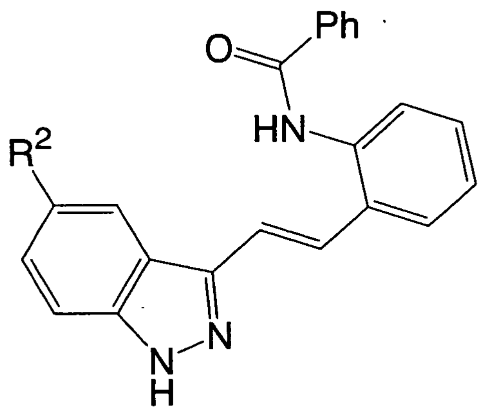

| Compound Nos. | $R^2$ |
|---|---|
| 25 | 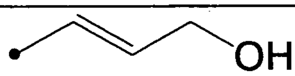 |
| 26 | 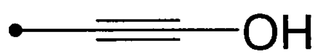 |

Table 3 (1)

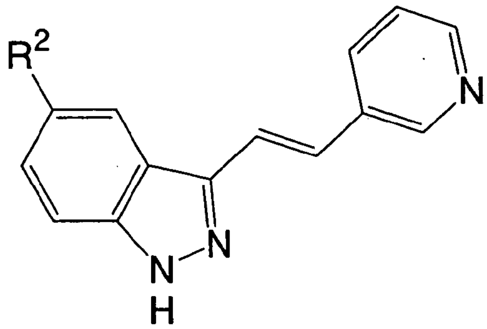

| Compound Nos. | $R^2$ |
|---|---|
| 2 | H |
| 3 | NHAc |
| 4 | NHBz |
| 5 | $NHCO(CH_2)_3CH_3$ |
| 6 | 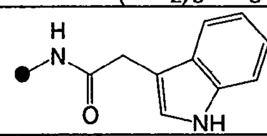 |
| 7 | 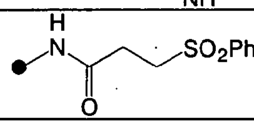 |
| 8 | 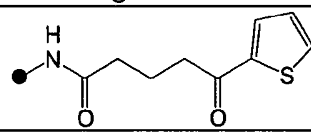 |
| 9 | 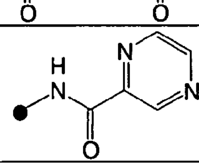 |
| 10 | 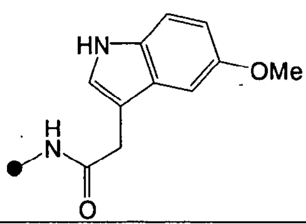 |
| 11 | 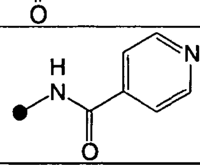 |
| 12 | 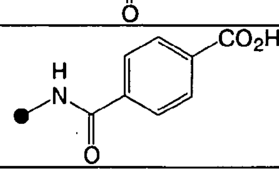 |

Table 3(2)

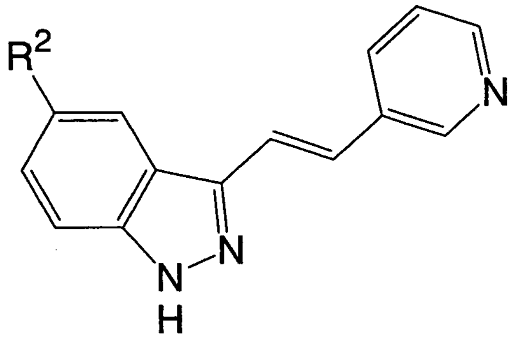

| Compound Nos. | $R^2$ |
|---|---|
| 13 | 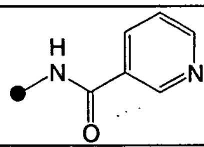 |
| 14 | 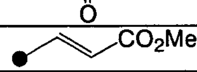 |
| 15 | 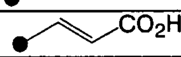 |
| 16 | 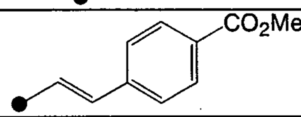 |
| 17 | 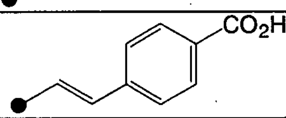 |
| 18 | 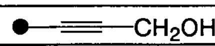 |
| 19 | $CO_2H$ |
| 20 | 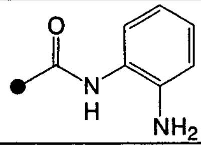 |
| 21 | 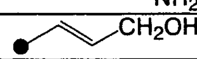 |
| 22 | 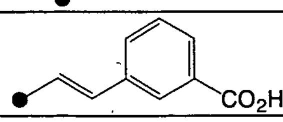 |

Table 4

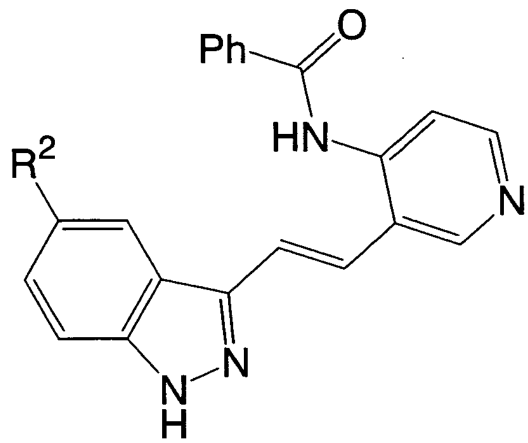

| Compound Nos. | $R^2$ |
|---|---|
| 23 | 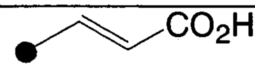 |
| 24 | 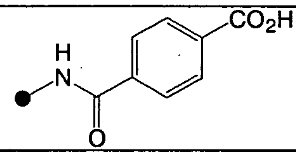 |

Compound (I) or a pharmaceutically acceptable salt thereof may be used either as it is or in various pharmaceutical forms depending upon the pharmacological effect, purpose of administration, etc. A pharmaceutical composition of the present invention can be manufactured by a uniform mixing of Compound (I) or a pharmaceutically acceptable salt thereof in an amount which is effective as an active ingredient with a pharmaceutically acceptable carrier. The carrier may be in a suitable form depending upon the form of the preparation for administration. It is preferred that the pharmaceutical composition is in a unit dosage form suitable for oral or parenteral administration such as injection.
In the manufacture of tablets, excipient such as lactose and mannitol, disintegrating agent such as starch, lubricant such as magnesium stearate, binder such as polyvinyl alcohol and hydroxypropyl cellulose, surfactant such as sucrose fatty acid ester and sorbitol fatty acid ester, etc. may be used in accordance with a conventional method. Tablets containing 1 to 200 mg of an active ingredient per tablet are preffered.
In the manufacture of injection preparations, water, physiological saline, plant oil such as olive oil and peanut oil, solvent such as ethyl oleate and propylene glycol, dissolving agent such as sodium benzoate, sodium salicylate and urethane, isotonizing agent such as salt and glucose, preservative such as phenol, cresol, p-hydroxybenzoate ester and chlorobutanol, antioxidant such as ascorbic acid and sodium pyrosulfite, etc. may be used by a conventional method.
Compound (I) or a pharmaceutically acceptable salt thereof can be administered either orally or parenterally by means of injection solution, etc. and, although its effective dose and administering times may vary depending upon dosage form, age, body weight and symptom of a patient, etc. In general, Compound (I) may preferably be administered in an amount of 0.1 to 50 mg/kg per day.
Activity of Compound (I) will be illustrated by the following Test Examples.

Test Example 1 JNK 3 Inhibitory Activity

A crude extract of COS-7 cells in which mouse JNK 3 was expressed was used as the transcription enzyme solution. Glutathione-S-transferase (GST)-JSAP 1 (JNK/SAPK associated protein 1) fused protein was expressed in E. coli and its crude extract was subjected to an affinity purification using a column of Glutathione Sepharose 4B. An

enzyme solution (3 μL), 50 μL of a reaction solution containing a test compound [in the reaction solution, (physiological saline)/(DMSO solution containing the test compound)(ratio by weight) was adjusted to 99/1], 50 μL of a substrate solution (final concentration: 3 μmol/L ATP), 0.75 μCi/ml[γ-$^{33}$P]-ATP and 66 μg/mL (1.5 μmol/L) of GST-JSAP1 fused protein reaction solution were incubated at 30°C for 15 minutes. The reaction was stopped by addition of ethanol whereupon the GST-JSAP1 fused protein was precipitated. The precipitated protein was filtered using a Filter Mate Harvester (Packard Instruments) and adsorbed to a Unifilter Plate GF/B. Incorporation of radiation activity from [γ-$^{33}$P] -ATP to the GST-JSAP1 fused protein was measured using TopCount-HTS (Packard Instruments) to calculate enzyme inhibition activity. The enzyme inhibitory activity was calculated by a Probit (L) method using a statistic analysis software SAS (Release 6.12; SAS Institute, Inc.) as the 50% enzyme activity inhibiting concentration ($IC_{50}$).

The results are shown in Table 5.

Table 5

| Compounds Nos. | JNK3 Inhibitory Activity ($IC_{50}$ / μM) |
|---|---|
| 1 | <0.5 |
| 2 | <0.5 |
| 6 | <0.5 |
| 7 | 1.2 |
| 9 | 0.55 |
| 11 | <0.5 |
| 12 | <0.5 |
| 14 | <0.5 |
| 15 | <0.5 |
| 17 | <0.5 |
| 18 | <0.5 |
| 19 | <0.5 |
| 21 | <0.5 |
| 23 | <0.5 |
| 24 | <0.5 |
| 25 | <0.5 |
| 26 | <0.5 |

Test Example 2 Apoptosis Suppressive Activity by Removal of Nerve Growth Factor (NGF) of Adrenal Cell Strain PC12 Differentiated by NGF

PC12 cells prepared in 6.0 × $10^4$ /mL by Roswell Park Memorial Institute's Medium (RPMI) 1640 medium containing 10% of horse serum, 5% of bovine fetal serum and penicillin/streptomycin were disposed in a 24-well multidish (NulgeNunc International; No. 143982) coated with polyethyleneimine in an amount of 0.5 mL for each well. After the cells were adhered to the dish, the medium was removed, an RPMI 1640 medium containing 0.5 mg of 1% horse serum, penicillin/streptomycin and 50 ng/mL of mouse nerve growth factor (NGF) was added and the cells were incubated in a carbon dioxide gas incubator at 37°C for 9 to 12 days, causing the cells to differentiate into a nerve cell form. A dilution series of DMSO solution of the test compound was prepared, and diluted with a medium containing 1% horse serum and penicillin/streptomycin and containing no NGF (final DMSO concentration: 0.1%). The medium was removed, washing was conducted twice using a medium containing 0.5 mL of the test compound, 1% horse serum, and penicillin/streptomycin, and containing no NGF, 0.5 mL of the above-medium was added once again to each well and then incubation was conducted in a carbon dioxide gas incubator at 37°C for 24 hours. The medium was removed, 0.2 mL of a medium containing 1% horse serum, penicillin/streptomycin, NGF and 1 mg/mL 3-(4,5-dimethyl-2-thiazolyl)-2,5-diphenyl-2H-tetrazolium bromide (MTT) containing no test compound, was added to each well and incubation was conducted in a carbon dioxide gas incubator at 37°C for 4 hours. DMSO (0.6 mL) was added to each well and 0.2 mL thereof was transferred to a 96-well multititer plate (Nulge Nunc International; No. 167008) and absorbances at 590 nm and 630 nm were measured using a microplate reader EL 3401 (Bio-Tek). The absorbance differential was calculated by subtracting the absorbance at 590 nm from the absorbance at 630 nm for each well. The apoptosis suppressive activity (%) of test compound were calculated by comparing the absorbance differential obtained from cells treated with test compounds of known concentration with the value in the cells from which no NGF was removed and the value in the cells from which NGF was removed. Here, the value in the cells from which no NGF was removed was set at 100% while the value in the cells from which NGF was removed was set at 0%.

The results are shown in Table 6.

Table 6

| Compounds Nos. | Apoptosis Suppressive Activity in 3 μmol/L of Test Compound (%) |
|---|---|
| 1 | 63 |
| 2 | 44 |
| 4 | 64 |
| 14 | 62 |

Best Mode for Carrying Out the Invention

As hereunder, the present invention will be illustrated in detail by the Examples.

In proton nuclear magnetic resonance spectrum ($^1$H-NMR) used in the Examples, there are some cases where exchanging hydrogen is not clearly observed depending upon compounds and measuring conditions. Incidentally, with regard to indication of multiplicity of signals, the commonly used notation is here used, although here br means an broad signal as judged by visual inspection.

Example 1 (E)-3-[2-(Benzamidopyridin-3-yl)vinyl]-(E)-5-(2-carboxyvinyl)-1H-indazole (Compound 23)

Step 1

In a similar manner to Reference Example 5, (E)-5-bromo-3-[2-(4-tert-butoxycarbonylaminopyridin-3-yl)-vinyl]-1H-indazole (271 mg, 72%) was obtained from (5-bromo-1H-indazole-3-ylmethyl)triphenhylphosphonium iodide (502 mg, 0.908 mmol), 4-tert-butoxycarbonylaminopyridine-3-carboxaldehyde (202 mg, 0.908 mmol) and potassium carbonate (377 mg, 2.72 mmol).

$^1$H-NMR (270 MHz, DMSO-$d_6$)δ; 1.48 (s, 9H), 7.47 (d, J = 16.5 Hz, 1H), 7.48-7.61 (m, 3H), 7.65 (d, J = 16.5 Hz, 1H), 8.34 (d, J = 5.6 Hz, 1H), 8.41 (s, 1H), 8.87 (s, 1H), 9.43 (brs, 1H), 13.40 (brs, 1H).

TOF-MS (m/z); 415[M+1]$^+$

Step 2

(E)-5-bromo-3-[2-(4-tert-butoxycarbonylaminopyridin-3-yl)vinyl]-1H-indazole (271 mg, 0.651 mmol) was suspended in tetrahydrofuran (7.5mL), sodium hydride (94 mg, 2.3 mmol) was added, the mixture was cooled at -78 °C and n-butyl lithium (a 1.56 mol/L hexane solution) (2.5 mL, 3.9 mmol) was added followed by stirring at the same temperature for 1 hour. To the reaction solution was added N,N-dimethylformamide (1.0 mL, 13 mmol) followed by stirring for additional 1 hour at the same temperature. Then the reaction solution was poured over ice and extracted with ethyl acetate. The organic layer was washed with saturated brine and dried over anhydrous magnesium sulfate, and the solvent was evaporated in *vacuo.* The residue was purified by silica gel column chromatography (chloroform/methanol/aqueous ammonia = 20/1/1) to give (E)-3-[2-(4-tert-butoxycarbonylaminopyridin-3-yl)vinyl]-1H -indazole-5-carboxaldehyde (132 mg, 56%).

$^1$H-NMR (270 MHz, DMSO-$d_6$)δ; 1.49 (s, 9H), 7.57 (d, J = 16.5 Hz, 1H), 7.66 (d, J = 5.5 Hz, 1H), 7.70 (d, J = 8.7 Hz, 1H), 7.78 (d, J = 16.5 Hz, 1H), 7.89 (d, J = 8.7 Hz, 1H), 8.38 (d, J = 5.6 Hz, 1H), 8.82 (s, 1H), 8.91 (s, 1H), 9.44 (brs, 1H), 10.07 (s, 1H), 13.67 (brs, 1H).

Step 3

To (E)-3-[2-(4-tert-butoxycarbonylaminopyridin-3-yl)-vinyl]-1H-indazole-5-carboxaldehyde (332 mg, 0.912 mmol) was added trifluoroacetic acid (3.77 mL, 49.1 mmol) followed by stirring at 40 °C for 2 hours. To the residue was added ice water, the mixture was made alkaline with a 10 mol/L aqueous solution of sodium hydroxide and the resulting precipitate was collected to obtain (E)-3-[2-(4-aminopyridin-3-yl)vinyl]-1H-indazole -5-carboxaldehyde (256 mg, quantitatively).

$^1$H-NMR (270 MHz, DMSO-$d_6$)δ; 6.43 (brs, 2H), 6.61 (d, J = 5.6 Hz, 1H), 7.44 (d, J = 16.5 Hz, 1H), 7.62 (d, J = 16.5 Hz, 1H), 7.67 (d, J = 8.9 Hz, 1H), 7.87 (dd, J = 1.2 Hz, 8.9 Hz, 1H), 7.96 (d, J = 5.6 Hz, 1H), 8.51 (s, 1H), 8.87 (s, 1H), 10.08 (s, 1H), 13.59 (brs, 1H).

Step 4

In a similar manner to Example 4 mentioned later, (E)-3-[2-(4-benzamidopyridin-3-yl)vinyl]-1H-indazole-5-carboxaldehyde (90 mg, 92%) was obtained from (E)-3-[2-(4-aminopyridin-3-yl)vinyl]-1H-indazole-5-carboxaldehyde (85 mg, 0.27 mmol), triethylamine (0.374 mL, 2.68 mmol) and benzoyl chloride (0.154 mL, 1.34 mmol).

$^1$H-NMR (270 MHz, DMSO-$d_6$)δ; 7.54-7.74 (m, 7H), 7.86 (d, J = 8.6 Hz, 1H), 8.04-8.07 (m, 2H), 8.50 (d, J = 5.6 Hz, 1H), 8.72 (s, 1H), 9.16 (s, 1H), 9.82 (s, 1H), 10.52 (brs, 1H), 13.63 (brs, 1H).

Step 5

In a similar manner to Example 14 mentioned later, (E)-3-[2-(4-benzamidopyridin-3-yl)vinyl]-(E)-5-(2-tert-butoxycarbonylvinyl)-1H-indazole (80 mg, quantitatively) was obtained from (E)-3-[2-(4-benzamidopyridin-3-yl)vinyl]-1H-indazole-5-carboxaldehyde (62 mg, 0.17 mmol) and tert-butyl triphenylphosphoranylideneacetate (146 mg, 0.388 mmol).

$^1$H-NMR (270 MHz, DMSO-$d_6$)δ; 1.51 (s, 9H), 6.59 (d, J = 15.8 Hz, 1H), 7.53-7.56 (m, 8H), 8.05-8.08 (m, 2H), 8.48-8.50 (m, 3H), 9.11 (s, 1H), 10.48 (brs, 1H), 13.36 (brs, 1H).

Step 6

In a similar manner to Step 3 of Example 1, Compound 23 (47 mg, 69%) was obtained by treating (E)-3-[2-(4-benzamidopyridin-3-yl)vinyl]-(E)-5-(2-tert-butoxycarbonylvinyl)-1H-indazole (78 mg, 0.17 mmol) with trifluoroacetic acid (0.60 mL, 7.8 mmol).

$^1$H-NMR (270 MHz, DMSO-$d_6$)δ; 6.60 (d, J = 16.2 Hz, 1H), 7.55-7.83 (m, 8H), 8.03-8.10 (m, 3H), 8.49 (s, 1H), 8.59 (d, J = 5.9 Hz, 1H), 9.19 (s, 1H), 10.70 (brs, 1H), 13.46 (brs, 1H).

TOF-MS (m/z); 411$[M+1]^+$

Example 2 (E)-3-[2-(4-Benzamidopyridin-3-yl)vinyl]-5-(4-carboxybenzamido)-1H-indazole (Compound 24)

Step 1

In a similar manner to Reference Example 5, (E)-3-[2-(4-benzamidopyridin-3-yl)vinyl]-5-nitro-1H-indazole (196 mg, 54%) was obtained from (5-nitro-1H-indazole-3-yl-methyl)triphenylphosphonium iodide (525 mg, 1.01 mmol), 4-benzamidopyridine-3-carboxaldehyde (214 mg, 0.946 mmol) and potassium carbonate (392 mg, 2.84 mmol).

$^1$H-NMR (270 MHz, DMSO-$d_6$)δ; 7.53-7.66 (m, 4H), 7.72 (d, J = 9.2 Hz, 1H), 7.74 (d, J = 16.5 Hz, 1H), 7.86 (d, J = 16.5 Hz, 1H), 8.05 (dt, J = 1.6 Hz, 6.9 Hz, 2H), 8.21 (dd, J = 2.0 Hz, 9.2 Hz, 1H), 8.49 (d, J = 5.3 Hz, 1H), 9.16 (s, 1H), 9.21 (d, J = 2.0 Hz, 1H).

TOF-MS (m/z); 384$[M-1]^-$

Step 2

In a similar manner to Reference Example 4 mentioned later, (E)-5-amino-3-[2-(4-benzamidopyridin-3-yl)vinyl]-1H -indazole (137 mg, 77%) was obtained by treating (E)-3-[2-(4-benzamidopyridin-3-yl)vinyl]-5-nitro-1H-indazole (194 mg, 0. 503 mmol) with hydrazine monohydrate (0.49 mL, 10 mmol) in the presence of 10% palladium-carbon (containing 50% of water; 194 mg).

$^1$H-NMR (270 MHz, DMSO-$d_6$)δ; 4.68 (brs, 2H), 6.79 (dd, J = 2.0 Hz, 8.9 Hz, 1H), 7.00 (brs, 1H), 7.24 (d, J = 8.9 Hz, 1H), 7.38 (d, J = 16.7 Hz, 1H), 7.48 (brs, 1H), 7.52-7.65 (m, 4H), 8.03 (dt, J = 1.7 Hz, 6.6 Hz, 2H), 8.43 (d, J = 5.6 Hz, 1H), 9.01 (s, 1H), 10.40 (brs, 1H), 12.76 (brs, 1H).

Step 3

In a similar manner to Example 4 mentioned later, (E)-3-[2-(4-benzamidopyridin-3-yl)vinyl]-5-(4-methoxycarbonylbenzamido)-1H-indazole (157 mg, 81%) was obtained from (E)-5-amino-3-[2-(4-benzamidopyridin-3-yl)-vinyl]-1H-indazole (133 mg, 0.374 mmol), triethylamine (0.42 mL, 3.0 mmol) and monomethyl chloroterephthalate (446 mg, 2.25 mmol).

TOF-MS (m/z); 518$[M+1]^+$

Step 4

In a similar manner to Step 2 of Example 12 mentioned later, Compound 24 (63 mg, 65%) was obtained by treating (E)-3-[2-(4-benzamidopyridin-3-yl)vinyl]-5-(4-methoxycarbonylbenzamido)-1H-indazole (100 mg, 0.193 mmol) with a 1 mol/L aqueous solution of sodium hydroxide (30 mL, 30 mmol).

$^1$H-NMR (270 MHz, DMSO-$d_6$)δ; 7.38-7.67 (m, 8H), 8.00 (dd, J = 1.7 Hz, 7.9 Hz, 2H), 8.03 (d, J = 8.6 Hz, 2H), 8.08 (d, J = 8.6 Hz, 2H), 8.46 (d, J = 5.6 Hz, 1H), 8.52 (s, 1H), 9.10 (s, 1H), 10.42 (brs, 1H), 10.44 (brs, 1H), 13.21 (brs, 1H).

TOF-MS (m/z); 504$[M+1]^+$

Example 3 (E)-5-Acetamido-3-[2-(3-pyridyl)vinyl]-1H-indazole (Compound 3)

Compound G (58 mg, 0.25 mmol) was dissolved in pyridine (0.60 mL) and acetic anhydride (0.070 mL, 0.74 mmol) was added thereto, followed by stirring at room temperature for 1 hour. Ice water was added to the reaction solution, the mixture was extracted with a mixed solvent of chloroform-methanol, the extract was washed with saturated brine and dried over anhydrous sodium sulfate and the solvent was evaporated *in vacuo.* The residue was purified by thin-layer chromatography (chloroform/methanol/aqueous ammonia = 9/1/1) followed by triturating with ethyl acetate to give Compound 3 (26 mg, 38%).

$^1$H-NMR (270 MHz, DMSO-$d_6$)δ; 2.07 (s, 3H), 7.37 (d, J = 16.7 Hz, 1H), 7.41 (dd, J = 4.5 Hz, 7.9 Hz, 1H), 7.49 (m, 2H), 7.63 (d, J = 16.7 Hz, 1H), 8.14 (d, J = 7.9 Hz, 1H), 8.37 (s, 1H), 8.46 (d, J = 4.5 Hz, 1H), 8.81 (s, 1H), 9.98 (s, 1H), 13.18 (brs, 1H).

TOF-MS (m/z); 279$[M+1]^+$

Example 4 (E)-5-Benzamido-3-[2-(3-pyridyl)vinyl]-1H-indazole (Compound 4)

Compound G (31 mg, 0.13 mmol) was dissolved in N-methylpyrrolidone (0.4 mL), triethylamine (0.072 mL, 0.52 mmol) and benzoyl chloride (0.045 mL, 0.39 mmol) were added thereto and the mixture was stirred at room temperature for 2 hours. Ice water was added to the reaction solution, the mixture was extracted with ethyl acetate, the extract was washed with saturated brine and dried over anhydrous sodium sulfate and the solvent was evaporated in *vacuo.* The residue was purified by thin-layer chromatography (chloroform/methanol = 9/1) followed by triturating with a mixed solvent of ethyl acetate/diisopropyl ether to give Compound 4 (13 mg, 29%).

$^1$H-NMR (270 MHz, DMSO-$d_6$)δ; 7.42 (d, J = 16.8 Hz, 1H), 7.43 (m, 1H), 7.54-7.75 (m, 6H), 8.01 (d, J = 7.6 Hz, 2H), 8.16 (dd, J = 1.5 Hz, 7.9 Hz, 1H), 8.47 (d, J = 4.8 Hz, 1H), 8.53 (s, 1H), 8.84 (brs, 1H), 10.32 (brs, 1H), 13.22 (brs, 1H).

TOF-MS (m/z) ; 341$[M+1]^+$

Example 5 (E)-5-Butaneamido-3-[2-(3-pyridyl)vinyl]-1H-indazole (Compound 5)

In a similar manner to Example 4, Compound 5 (14 mg, 33%) was obtained from Compound G (30 mg, 0.13 mmol), triethylamine (0.072 mL, 0.52 mmol) and valeryl chloride (0.046 mL, 0.39 mmol).

$^1$H-NMR (270 MHz, DMSO-$d_6$)δ; 0.91 (t, J = 7.3 Hz, 3H), 1.35 (m, 2H), 1.61 (m, 2H), 2.33 (t, J = 7.4 Hz, 2H), 7.36 (d, J = 16.7 Hz, 1H), 7.41 (dd, J = 4.6 Hz, 8.3 Hz, 1H), 7.49 (m, 2H), 7.63 (d, J = 16.7 Hz, 1H), 8.14 (d, J = 8.3 Hz, 1H), 8.40 (s, 1H), 8.46 (dd, J = 1.7 Hz, 4.6 Hz, 1H), 8.81 (d, J = 1.7 Hz, 1H), 9.90 (brs, 1H), 13.16 (brs, 1H).

TOF-MS (m/z); 321$[M+1]^+$

Example 6 (E)-5-(3-Indolyl)acetamide-3-[2-(3-pyridyl) vinyl]-1H-indazole (Compound 6)

Tetrahydrofuran (0.8 mL) was added to a mixture of Compound G (30 mg, 0.13 mmol), indole-3-acetic acid (33 mg, 0.19 mmol), 1-hydroxybenzotriazole monohydrate (29 mg, 0.19 mmol) and 1-ethyl-3-(3-dimethylaminopropyl) carbodiimide hydrochloride (36 mg, 0.19 mmol) followed by stirring at room temperature for 2.5 hours. Water was added to the reaction solution, the mixture was extracted with ethyl acetate and the extract was dried over anhydrous magnesium sulfate, purified by thin-layer chromatography (chloroform/methanol = 9/1) followed by triturating with ethyl acetate to give Compound 6 (16 mg, 32%).

$^1$H-NMR (270 MHz, DMSO-$d_6$)δ; 3.76 (s, 2H), 6.98 (t, J = 6.9 Hz, 1H), 7.06 (dd, J = 6.9 Hz, 7.9 Hz, 1H), 7.28-7.65 (m, 8H), 8.12 (d, J = 8.6 Hz, 1H), 8.40 (s, 1H), 8.44 (brs, 1H), 8.79 (brs, 1H), 10.15 (brs, 1H), 10.91 (brs, 1H), 13.17 (brs, 1H).

TOF-MS (m/z); 394$[M+1]^+$

Example 7 (E)-5-[3-(Phenylsulfonyl)propaneamido]-3-[2-(3-pyridyl)vinyl]-1H-indazole (Compound 7)

In a similar manner to Example 6, Compound 7 (25 mg, 46%) was obtained from Compound G (30 mg, 0.13 mmol), 3-(phenylsulfonyl)propionic acid (41 mg, 0.19 mmol), 1-hydroxybenzotriazole monohydrate (29 mg, 0.19 mmol) and 1-ethyl-3-(3-dimethylaminopropyl)carbodiimide hydrochloride (36 mg, 0.19 mmol).
$^1$H-NMR (270 MHz, DMSO-$d_6$)δ; 2.71 (t, J = 7.6 Hz, 2H), 3.65 (t, J = 7.6 Hz, 2H), 7.33 (d, J = 16.7 Hz, 1H), 7.39-7.43 (m, 2H), 7.49 (d, J = 8.9 Hz, 1H), 7.62 (d, J = 16.7 Hz, 1H), 7.63-7.75 (m, 3H), 7.92-7.95 (m, 2H), 8.13 (brd, J = 8.3 Hz, 1H), 8.30 (s, 1H), 8.46 (dd, J = 1.7 Hz, 4.8 Hz, 1H), 8.80 (d, J = 1.7 Hz, 1H), 10.12 (brs, 1H), 13.19 (brs, 1H).
TOF-MS (m/z); 433[M+1]$^+$

Example 8 (E)-5-[5-Oxo-5-(2-thienyl)pentanamido]-3-[2-(3-pyridyl)vinyl]-1H-indazole (Compound 8)

In a similar manner to Example 6, Compound 8 (12 mg, 23%) was obtained from Compound G (30 mg, 0.13 mmol), 5-oxo-5-(2-thienyl)valeric acid (38 mg, 0.19 mmol), 1-hydroxybenzotriazole monohydrate (29 mg, 0.19 mmol) and 1-ethyl-3-(3-dimethylaminopropyl)carbodiimide hydrochloride (36 mg, 0.19 mmol).
$^1$H-NMR (270 MHz, DMSO-$d_6$)δ; 1.97 (m, 2H), 2.43 (t, J = 7.4 Hz, 2H), 3.05 (t, J = 7.1 Hz, 2H), 7.23 (t, J = 4.3 Hz, 1H), 7.36 (d, J = 16.7 Hz, 1H), 7.41 (m, 1H), 7.49 (m, 2H), 7.63 (d, J = 16.7 Hz, 1H), 7.96 (d, J = 4.3 Hz, 1H), 7.98 (d, J = 4.3 Hz, 1H), 8.14 (d, J = 7.9 Hz, 1H), 8.40 (s, 1H), 8.46 (d, J = 3.3 Hz, 1H), 8.80 (s, 1H), 9.96 (brs, 1H), 13.17 (brs, 1H).
TOF-MS (m/z); 417[M+1]$^+$

Example 9 (E)-5-(Pyrazinecarboxamido)-3-[2-(3-pyridyl) vinyl]-1H-indazole (Compound 9)

In a similar manner to Example 6, Compound 9 (6.4 mg, 15%) was obtained from Compound G (30 mg, 0.13 mmol), 2-pyrazinecarboxylic acid (24 mg, 0.19 mmol), 1-hydroxybenzotriazole monohydrate (29 mg, 0.19 mmol) and 1-ethyl-3-(3-dimethylaminopropyl)carbodiimide hydrochloride (36 mg, 0.19 mmol).
$^1$H-NMR (270 MHz, DMSO-$d_6$)δ; 7.42 (m, 1H), 7.45 (d, J = 16.3 Hz, 1H), 7.57 (d, J = 8.6 Hz, 1H), 7.67 (d, J = 16.3 Hz, 1H), 7.94 (d, J = 8.6 Hz, 1H), 8.17 (d, J = 7.6 Hz, 1H), 8.47 (d, J = 4.0 Hz, 1H), 8.66 (s, 1H), 8.84 (m, 2H), 8.94 (d, J = 2.6 Hz, 1H), 9.33 (s, 1H), 10.79 (brs, 1H), 13.26 (brs, 1H).
TOF-MS (m/z); 343[M+1]$^+$

Example 10 (E)-5-(5-Methoxyindol-3-yl)acetamido-3-[2-(3-pyridyl)vinyl]-1H-indazole (Compound 10)

In a similar manner to Example 6, Compound 10 (21 mg, 39%) was obtained from Compound G (30 mg, 0.13 mmol), 5-methoxyindole-3-acetic acid (39mg, 0.19 mmol), 1-hydroxybenzotriazole hydrate (29 mg, 0.19 mmol) and 1-ethyl-3-(3-dimethylaminopropyl)carbodiimide hydrochloride (36 mg, 0.19 mmol).
$^1$H-NMR (270 MHz, DMSO-$d_6$)δ; 3.72 (s, 2H), 3.73 (s, 3H), 6.71 (dd, J = 2.3 Hz, 8.9 Hz, 1H), 7.16 (d, J = 2.3 Hz, 1H), 7.23 (d, J = 8.9 Hz, 1H), 7.24 (s, 1H), 7.34 (d, J = 16.7 Hz, 1H), 7.40 (dd, J = 5.0 Hz, 7.9 Hz, 1H), 7.51 (m, 2H), 7.62 (d, J = 16.7 Hz, 1H), 8.12 (d, J = 7.9 Hz, 1H), 8.41 (s, 1H), 8.45 (dd, J = 1.5 Hz, 5.0 Hz, 1H), 8.79 (d, J = 1.5 Hz, 1H), 10.15 (brs, 1H), 10.76 (brs, 1H), 13.18 (brs, 1H).
TOF-MS (m/z); 424[M+1]$^+$

Example 11 (E)-5-(4-Pyridinecarboxamido)-3-[2-(3-pyridyl) vinyl]-1H-indazole (Compound 11)

In a similar manner to Example 6, Compound 11 (8.7 mg, 20%) was obtained from Compound G (30 mg, 0.13 mmol), isonicotinic acid (24 mg, 0.19 mmol), 1-hydroxybenzotriazole monohydrate (29 mg, 0.19 mmol) and 1-ethyl-3-(3-dimethylaminopropyl)carbodiimide hydrochloride (36 mg, 0.19 mmol).
$^1$H-NMR (270 MHz, DMSO-$d_6$)δ; 7.41 (dd, J = 4.6 Hz, 7.6 Hz, 1H), 7.43 (d, J = 16.7 Hz, 1H), 7.57 (d, J = 8.9 Hz, 1H), 7.68 (d, J = 16.7 Hz, 1H), 7.71 (d, J = 8.9 Hz, 1H), 7.91 (d, J = 5.9 Hz, 2H), 8.16 (d, J = 7.6 Hz, 1H), 8.46 (dd, J = 1.8 Hz, 4.6 Hz, 1H), 8.54 (s, 1H), 8.80 (d, J = 5.9 Hz, 2H), 8.84 (d, J = 1.8 Hz, 1H), 10.59 (brs, 1H), 13.27 (brs, 1H).
TOF-MS (m/z); 342[M+1]$^+$

Example 12 (E)-5-(4-Carboxybenzamido)-3-[2-(3-pyridyl) vinyl]-1H-indazole (Compound 12)

Step 1

In a similar manner to Example 4, (E)-5-(4-methoxycarbonylbenzamido)-3-[2-(3-pyridyl)vinyl]-1H-indazole (132 mg, 72%) was obtained from Compound G (108 mg, 0.458 mmol), triethylamine (0.51 mL, 3.7 mmol) and monomethyl chloroterepthalate (546 mg, 2.75 mmol).

$^1$H-NMR (270 MHz, DMSO-$d_6$)δ; 3.90 (s, 3H), 7.41 (m, 1H), 7.42 (d, J = 17.0 Hz, 1H), 7.56 (d, J = 8.9 Hz, 1H), 7.68 (d, J = 17.0 Hz, 1H), 7.73 (m, 1H), 8.09-8.18 (m, 5H), 8.46 (dd, J = 1.6 Hz, 4.6 Hz, 1H), 8.53 (s, 1H), 8.83 (d, J = 2.0 Hz, 1H), 10.51 (brs, 1H), 13.25 (brs, 1H).

TOF-MS (m/z); 399[M+1]$^+$

Step 2

(E)-5-(4-Methoxycarbonylbenzamido)-3-[2-(3-pyridyl)-vinyl]-1H-indazole (129 mg, 0.324 mmol) was dissolved in a mixed solvent of tetrahydrofuran (10 mL) and methanol (14 mL), then a 1 mol/L aqueous solution of sodium hydroxide (5.0 mL, 5. 0 mmol) and water (3 mL) were added and the mixture was stirred at room temperature for 2 hours. The organic solvents were evaporated *in vacuo,* the residue was adjusted to pH 4 with 1 mol/L hydrochloric acid and the resulting precipitate was filtered and triturated under heating with a mixed solvent of ethanol-water to give Compound 12 (77 mg, 62%)

$^1$H-NMR (270 MHz, DMSO-$d_6$)δ; 7.42 (dd, J = 5.3 Hz, 7.9 Hz, 1H), 7.43 (d, J = 16.8 Hz, 1H), 7.57 (d, J = 8.6 Hz, 1H), 7.69 (d, J = 16.8 Hz, 1H), 7.74 (d, J = 8.6 Hz, 1H), 8.07-8.14 (m, 4H), 8.17 (dt, J = 1.8 Hz, 7.9 Hz, 1H), 8.47 (dd, J = 1.8 Hz, 4.6 Hz, 1H), 8.54 (s, 1H), 8.55 (d, J = 1.8 Hz, 1H), 10.49 (brs, 1H), 13.26 (brs, 1H).

TOF-MS (m/z); 385[M+1]$^+$

Example 13 (E)-5-(3-Pyridinecarboxamido)-3-[2-(3-pyridyl) vinyl]-1H-indazole (Compound 13)

In a similar manner to Example 6, Compound 13 (8.6 mg, 20%) was obtained from Compound G (30 mg, 0.13 mmol), nicotinic acid (23 mg, 0.19 mmol), 1-hydroxybenzotriazole (29 mg, 0.19 mmol) and 1-ethyl-3-(3-dimethylaminopropyl)-carbodiimide hydrochloride (36 mg, 0.19 mmol).

$^1$H-NMR (270 MHz, DMSO-$d_6$)δ; 7.416 (dd, J = 4.6 Hz, 7.9 Hz, 1H), 7.424 (dd, J = 16.7 Hz, 1H), 7.57 (d, J = 8.7 Hz, 1H), 7.58 (dd, J = 4.7 Hz, 8.1 Hz, 1H), 7.67 (d, J = 16.7 Hz, 1H), 7.71 (dd, J = 1.8 Hz, 8.7 Hz, 1H), 8.16 (d, J = 7.9 Hz, 1H), 8.34 (dt, J = 1.8 Hz, 8.1 Hz, 1H), 8.46 (dd, J = 1.5 Hz, 4.6 Hz, 1H), 8.53 (brs, 1H), 8.77 (dd, J = 1.8 Hz, 4.7 Hz, 1H), 8.83 (d, J = 1.5 Hz, 1H), 9.16 (d, J = 1.8 Hz, 1H), 10.52 (brs, 1H), 13.26 (brs, 1H).

TOF-MS (m/z); 342[M+1]$^+$

Example 14 (E)-5-(2-Methoxycarbonylvinyl)-(E)-3-[2-(3-pyridyl)vinyl]-1H-indazole (Compound 14)

Toluene (2 mL) was added to a mixture of Compound F (32 mg, 0.14 mmol) and methyl triphenylphosphoranylideneacetate (78 mg, 0.23 mmol) followed by heating to reflux for 30 minutes. The reaction solution was purified by thin-layer chromatography (chloroform/methanol = 9/1) to give Compound 14 (36 mg, 88%).

$^1$H-NMR (270 MHz, DMSO-$d_6$)δ; 3.74 (s, 3H), 6.70 (d, J = 16.1 Hz, 1H), 7.42 (dd, J = 4.6 Hz, 8.3 Hz, 1H), 7.56 (d, J = 8.7 Hz, 1H), 7.61 (d, J = 16.5 Hz, 1H), 7.69 (d, J = 16.5 Hz, 1H), 7.81 (dd, J = 1.2 Hz, 8.7 Hz, 1H), 7.88 (d, J = 16.1 Hz, 1H), 8.17 (dt, J = 1.7 Hz, 8.3 Hz, 1H), 8.47 (dd, J = 1.7 Hz, 4.6 Hz, 1H), 8.59 (brs, 1H), 8.91 (d, J = 1.7 Hz, 1H).

TOF-MS (m/z); 306[M+1]$^+$

Example 15 (E)-5-(2-Carbonxyvinyl)-(E)-3-[2-(3-pyridyl)vinyl]-1H-indazole (Compound 15)

In a manner similar to Step 2 of Example 12, Compound 15 (8.6 mg, 85%) was obtained by treating Compound 14 (10 mg, 0.031 mmol) with 1 mol/L aqueous solution of sodium hydroxide (0.22 mL, 0.22 mmol).

$^1$H-NMR (270 MHz, DMSO-$d_6$)δ; 6.58 (d, J = 15.8 Hz, 1H), 7.44 (dd, J = 4.0 Hz, 8.1 Hz, 1H), 7.56 (d, J = 8.3 Hz, 1H), 7.61 (d, J = 16.9 Hz, 1H), 7.70 (d, J = 16.9 Hz, 1H), 7.78 (d, J = 8.3 Hz, 1H), 7.81 (d, J = 15.8 Hz, 1H), 8.20 (brd, J = 8.1 Hz, 1H), 8.48 (brd, J = 4.0 Hz, 1H), 8.55 (s, 1H), 8.92 (s, 1H), 12.28 (brs, 1H), 13.39 (brs, 1H).

TOF-MS (m/z); 292[M+1]$^+$

Example 16 (E)-5-[2-(4-Methoxycarbonylphenyl)vinyl]-(E)-3-[2-(3-pyridyl)vinyl]-1H-indazole (Compound 16)

In a similar manner to Reference Example 5, Compound 16 (16 mg, 21%) was obtained from Compound F (46 mg, 0.20 mmol), (4-methoxycarbonylphenylmethyl)triphenylphosphonium bromide (128 mg, 0.260 mmol) and potassium carbonate (38 mg, 0.27 mmol).

$^1$H-NMR (270 MHz, DMSO-$d_6$)δ; 3.85 (s, 3H), 7.39 (d, J = 16.2 Hz, 1H), 7.47 (dd, J = 4.6 Hz, 8.3 Hz, 1H), 7.58 (d, J = 8.9 Hz, 1H), 7.59 (d, J = 16.2 Hz, 1H), 7.61 (d, J = 16.7 Hz, 1H), 7.72 (d, J = 16.7 Hz, 1H), 7.75 (d, J = 8.4 Hz, 2H), 7.79 (dd, J = 1.0 Hz, 8.9 Hz, 1H), 7.97 (d, J = 8.4 Hz, 2H), 8.24 (brdd, J = 2.0 Hz, 8.3 Hz, 1H), 8.43 (brs, 1H),

8.50 (dd, J = 1.3 Hz, 4.6 Hz, 1H), 8.94 (d, J = 2.0 Hz, 1H), 13.33 (brs, 1H).
TOF-MS (m/z); 382[M+1]$^+$

Example 17 (E)-5-[2-(4-Carboxyphenyl)vinyl]-(E)-3-[2-(3-pyridyl)vinyl]-1H-indazole (Compound 17)

In a similar manner to Step 2 of Example 12, Compound 16 (16 mg, 0.041 mmol) was treated with a 1 mol/L aqueous solution of sodium hydroxide (0.67 mL, 0.67 mmol) to give Compound 17 (12 mg, 82%)
$^1$H-NMR (270 MHz, DMSO-$d_6$)δ; 3.85 (s, 3H), 7.39 (d, J = 16.2 Hz, 1H), 7.47 (dd, J = 4.6 Hz, 8.3 Hz, 1H), 7.58 (d, J = 8.9 Hz, 1H), 7.59 (d, J = 16.2 Hz, 1H), 7.61 (d, J = 16.7 Hz, 1H), 7.72 (d, J = 16.7 Hz, 1H), 7.75 (J = 8.4 Hz, 2H), 7. 79 (dd, J = 1.0 Hz, 8.9 Hz, 1H), 7.97 (d, J = 8.4 Hz, 2H), 8.24 (brdd, J = 2.0 Hz, 8.3 Hz, 1H), 8.43 (brs, 1H), 8.50 (dd, J = 1.3 Hz, 4.6 Hz, 1H), 8.94 (d, J = 2.0 Hz, 1H), 13.33 (brs, 1H).
TOF-MS (m/z); 368[M+1]$^+$

Example 18 (E)-5-(3-hydroxy-1-propyn-1-yl)-3-[2-(3-pyridyl)vinyl]-1H-indazole (Compound 18)

Step 1

Compound E (2.02 g, 6.73 mmol) was suspended in dichloromethane (80 mL) and then triethylamine (2.8 mL, 20 mmol) and benzenesulfonyl chloride (1.94 mL, 15.1 mmol) were added thereto followed by stirring at room temperature for 12 hours. Water was added to the reaction solution, the mixture was made alkaline with potassium carbonate and extracted with chloroform, the organic layer was dried over anhydrous magnesium sulfate and the solvent was evaporated *in vacuo.* The residue was purified by silica gel column chromatography (hexane/ethyl acetate = 2/1) and further triturated with methanol to give 1-benzenesulfonyl compound (2.08 g, 70%).
$^1$H-NMR (270 MHz, $CDCl_3$)δ; 7.32 (d, J = 16.7 Hz, 1H), 7.34 (dd, J = 4.8 Hz, 8.1 Hz, 1H), 7.48 (m, 2H), 7.55 (d, J = 16.7 Hz, 1H), 7.59 (m, 1H), 7.68 (dd, J = 1.8 Hz, 8.9 Hz, 1H), 7.89 (dt, J = 1.8 Hz, 8.1 Hz, 1H), 8.00 (m, 2H), 8.06 (dd, J = 0.5 Hz, 1.8 Hz, 1H), 8.13 (d, J = 8.9 Hz, 1H), 8.57 (dd, J = 1.8 Hz, 4.8 Hz, 1H), 8.79 (d, J = 1.8 Hz, 1H).
TOF-MS (m/z); 441[M+1]$^+$

Step 2

Triethylamine (0.60 mL, 4.3 mmol) and propargyl alcohol (0.007 mL, 0.1 mol) bubbled with argon gas were added to the benzenesulfonyl compound (49 mg, 0.11 mmol) obtained in Step 1, dichlorobis (triphenylphosphine) palladium (7.6 mg, 0.011 mmol) and cuprous iodide (2.3 mg, 0.012 mmol) and the mixture was stirred at 100 °C for 1 hour. After cooling to room temperature, ethyl acetate, water and tetrahydrofuran were added thereto, the organic layer was washed with saturated brine and dried over anhydrous magnesium sulfate and the solvents were evaporated in *vacuo.* The residue was purified by thin-layer chromatography (chloroform/methanol = 9/1) to give 1-benzenesulfonyl-(E)-5-(3-hydroxy-1-propyn-1-yl)-3-[2-(3-pyridyl)vinyl]-1H-indazole (9.8 mg, 21%).
$^1$H-NMR (270 MHz, $CDCl_3$)δ; 4.55 (s, 2H), 7.32 (d, J = 16.8 Hz, 1H), 7.33 (dd, J = 4.8 Hz, 7.9 Hz, 1H), 7.41-7.63 (m, 5H), 7.88 (dt, J = 1.7 Hz, 7.9 Hz, 1H), 7.98-8.03 (m, 3H), 8.17 (dd, J = 0.7 Hz, 8.7 Hz, 1H), 8.55 (brd, J = 4.8 Hz, 1H), 8.78 (brs, 1H).

Step 3

Methanol (1 mL) was added to 1-benzenesulfonyl-(E)-5-(3-hydroxy-1-propyn-1-yl)-3-[2-(3-pyridyl)vinyl]-1H-indazole (21 mg, 0.051 mmol), and while being heated under reflux a 1 mol/L aqueous solution of sodium hydroxide (0.5 mL, 0.5 mmol) was added, and the mixture was heated under reflux for 13 hours. After cooling to room temperature, ethyl acetate, water and tetrahydrofuran were added thereto, the organic layer was washed with saturated brine and dried over anhydrous magnesium sulfate and the solvent was evaporated *in vacuo.* The residue was purified by thin-layer chromatography (chloroform/methanol = 9/1) to give Compound 18 (5.0 mg, 36%).
$^1$H-NMR (270 MHz, DMSO-$d_6$)δ; 4.33 (d, J = 5.6 Hz, 2H), 5.32 (t, J = 5.6 Hz, 1H), 7.38-7.43 (m, 2H), 7.53 (d, J = 16.5 Hz, 1H), 7.55 (m, 1H), 7.70 (d, J = 16.5 Hz, 1H), 8.18 (brd, J = 7.9 Hz, 1H), 8.34 (s, 1H), 8.46 (brd, J = 3.6 Hz, 1H), 8.90 (s, 1H), 13.38 (brs, 1H).
TOF-MS (m/z); 276[M+1]$^+$

Example 19 (E)-3-[2-(3-Pyridyl)vinyl]-1H-indazole-5-carboxylic acid (Compound 19)

Compound E (1.04 g, 3.47 mmol) was dissolved in tetrahydrofuran (50 mL), sodium hydride (152 mg, 3.81 mmol) was added thereto at 0 °C, the mixture was stirred at the same temperature for 20 minutes and cooled at -78

°C and a 1.56 mol/L hexane solution of n-butyl lithium (3.3 mL, 5.1 mmol) was added followed by stirring at the same temperature for 1 hour. The reaction solution was bubbled with carbon dioxide gas, stirred at the same temperature for additional 30 minutes and the temperature was raised to room temperature and dry ice was added thereto followed by stirring for additional 30 minutes. After water and ethyl acetate were added to the reaction solution, the aqueous layer was made alkaline with a 10 mol/L aqueous solution of sodium hydroxide and an aimed product was extracted from the organic layer with diluted aqueous solution of sodium hydroxide. The aqueous layers were combined, washed with ethyl acetate and acidified with 6 mol/L hydrochloric acid and the resulting precipitate was filtered to give Compound 19 (655 mg, 71%).

$^1$H-NMR (270 MHz, DMSO-$d_6$)δ; 7.43 (dd, J = 4.9 Hz, 8.0 Hz, 1H), 7.55 (d, J = 16.6 Hz, 1H), 7.62 (d, J = 8.8 Hz, 1H), 7.84 (d, J = 16.6 Hz, 1H), 7.96 (dd, J = 1.4 Hz, 8.8 Hz, 1H), 8.24 (dt, J = 1.8 Hz, 8.0 Hz, 1H), 8.49 (dd, J = 1.8 Hz, 4.9 Hz, 1H), 8.81 (brs, 1H), 8.92 (d, J = 1.8 Hz, 1H), 12.86 (brs, 1H), 13.52 (brs, 1H).

TOF-MS (m/z); 266[M+1]$^+$

Example 20 N-(2-Aminophenyl)-(E)-3-[2-(3-pyridyl)vinyl] -1H-indazole-5-carboxamide (Compound 20)

Triphenylphosphine oxide (207 mg, 0.745 mmol) was added to dichloromethane (1.5 mL), then trifluoromethanesulfonic acid anhydride (0.061 mL, 0.37 mmol) was added at 0 °C and the mixture was stirred at the same temperature for 30 minutes. To the reaction solution was added suspension of Compound 19 (39 mg, 0.15 mmol) and 1, 2 -phenylenediamine (16 mg, 0.15 mmol) in tetrahydrofuran (2 mL) and temperature of the mixture was raised to room temperature followed by stirring for 1 hour. To the reaction solution was added a saturated aqueous solution of sodium hydrogen carbonate, the mixture was extracted with chloroform, the organic layer was dried over anhydrous magnesium sulfate and the solvent was evaporated in *vacuo.* The residue was purified by preparative thin-layer chromatography (chloroform/methanol/aqueous ammonia = 9/1/1) to give Compound 20 (8.3 mg, 16%).

$^1$H-NMR (270 MHz, DMSO-$d_6$)δ; 4.95 (brs, 2H), 6.62 (dt, J = 1.4 Hz, 7.9 Hz, 1H), 6.80 (dd, J = 1.4 Hz, 7.9 Hz, 1H), 6.98 (dt, J = 1.4 Hz, 7.9 Hz, 1H), 7.21 (dd, J = 1.4 Hz, 7.9 Hz, 1H), 7.42 (dd, J = 4.7 Hz, 8.1 Hz, 1H), 7.63 (d, J = 8.7 Hz, 1H), 7.64 (d, J = 16.8 Hz, 1H), 7.75 (d, J = 16.8 Hz, 1H), 8.00 (dd, J = 1.2 Hz, 8.7 Hz, 1H), 8.19 (dt, J = 1.7 Hz, 8.1 Hz, 1H), 8.48 (dd, J = 1.7 Hz, 4.7 Hz, 1H), 8.85 (brs, 1H), 8.89 (d, J = 1.7 Hz, 1H), 9.75 (brs, 1H), 13.47 (brs, 1H).

TOF-MS (m/z); 354[M-1]$^-$

Example 21 (E)-5-(3-Hydroxy-1-propen-1-yl)-3-[2-(3-pyridyl)vinyl]-1H-indazole .(Compound 21)

Compound 14 (201.3 mg, 0.66 mmol) obtained in Example 14 was dissolved in dichloromethane (40 mL), 2.6 mL (2.63 mmol) of diisobutyl aluminum hydride (1.0 mol/L toluene solution) was added thereto at -78 °C and the mixture was stirred at the same temperature for 1 hour, gradually warmed up to room temperature and stirred for additional 1 hour. The reaction was stopped by addition of water, an aqueous solution of sodium potassium (+)-tartrate and chloroform were added thereto and yellow solid separated out therefrom was obtained by means of filtration with suction and washed with water. The filtrate was extracted with chloroform, the organic layer was washed with saturated brine and dried over anhydrous sodium sulfate and the solvent was evaporated *in vacuo.* The residue and the yellow solid were purified by silica gel column chromatography (chloroform/methanol = 20/1) to give Compound 21 (138.4 mg, 76%).

$^1$H-NMR (270 MHz, DMSO-$d_6$)δ; 4.12-4.18 (m, 2H), 4.86 (dd, J = 5.2 Hz, 5.4 Hz, 1H), 6.43 (dt, J = 5.2 Hz, 15.9 Hz, 1H), 6.73 (d, J = 15.9 Hz, 1H), 7.41 (dd, J = 4.8 Hz, 8.1 Hz, 1H), 7.45-7.62 (m, 3H), 7.68 (d, J = 16.8 Hz, 1H), 8.12-8.22 (m, 2H), 8.46 (d, J = 4.8 Hz, 1H), 8.90 (s, 1H).

TOF-MS (m/z); 278[M+1]$^+$

Example 22 (E)-5-[2-(3-Carboxyphenyl)vinyl]-(E)-3-[2-(3-pyridyl)vinyl]-1H-indazole (Compound 22)

Step 1

Tetrahydrofuran (10 mL) and 1,8-diazabicyclo[5.4.0]-undec-7-ene (0.15 mL, 1.0 mmol) were added to a mixture of Compound F (55 mg, 0.22 mol), (3-methoxycarbonylphenylmethyl)triphenylphosphonium bromide (142 mg, 0.289 mmol) and lithium iodide (40 mg, 0.30 mmol) followed by heating under reflux for one night.

After cooling to room temperature, water was added to the reaction solution, the mixture was extracted with ethyl acetate, the organic layer was washed with water and saturated brine successively and dried over anhydrous magnesium sulfate and the solvent was evaporated *in vacuo.* The residue was purified by thin-layer chromatography (chloroform/methanol/aqueous ammonia = 200/10/1) to give (E)-5-[2-(3-methoxycarbonylphenyl)vinyl]-(E)-3-[2-(3-pyridyl)vinyl]-1H-indazole (24 mg, 29%).

$^1$H-NMR (270 MHz, $CDCl_3$)δ; 3.96 (s; 3H), 7.16 (d, J = 16.3 Hz, 1H), 7.34-7.58 (m, 6H), 7.69-7.75 (m, 2H), 7.92 (dt, J = 1.3 Hz, 6.4 Hz, 1H), 8.01 (dt, J = 1.7 Hz, 7.9 Hz, 1H), 8.06 (brs, 1H), 8.23 (s, 1H), 8.46 (brd, J = 3.6 Hz,

1H), 8.77 (brd, J = 1.7 Hz, 1H), 10.08 (brs, 1H).

TOF-MS (m/z); $382[M+1]^+$

Step 2

In a similar manner to step 2 of Example 12, (E) - 5 - [2 - (3 - methoxycarbonylphenyl) vinyl] - (E) - 3 - [2 - (3-pyridyl)vinyl]-1H-indazole (24 mg, 0.063 mmol) was treated with a 1 mol/L aqueous solution of sodium hydroxide (1.0 mL, 1.0 mmol) to give Compound 22 (12 mg, 52%).

$^1$H-NMR (270 MHz, DMSO-$d_6$)$\delta$; 7.40 (d, J = 16.9 Hz, 1H), 7.41-7.61 (m, 5H), 7.70 (d, J = 16.9 Hz, 1H), 7.78-7.87 (m, 3H), 8.19 (brd, J = 8.3 Hz, 1H), 8.20 (s, 1H), 8.42 (s, 1H), 8.48 (brd, J = 4.5 Hz, 1H), 8.92 (s, 1H), 13.29 (brs, 1H).

TOF-MS (m/z); $368[M+1]^+$

Example 23 (E)-3-[2-(2-Benzoylaminophenyl)vinyl]-(E)-5-(3-hydroxy-1-propen-1-yl)-1H-indazole (Compound 25)

Step 1

In a similar manner to Example 14, (E)-3-[2-(2-benzoylaminophenyl)vinyl]-5-bromo-1H-indazole (1.39 g, 74%) was obtained from (5-bromo-1H-indazol-3-ylmethyl)triphenylphosphonium bromide (2.48 g, 4.49 mmol), N-(2-formylphenyl)benzamide (1.02 g, 4.53 mmol) and potassium carbonate (1.89 g, 13.67 mmol).

$^1$H-NMR (270 MHz, DMSO-$d_6$)$\delta$; 7.34-7.43 (m, 3H), 7.46 (dd, J = 1.6 Hz, 8.7 Hz, 1H), 7.51-7.65 (m, 6H), 7.99-8.02 (m, 1H), 8.08 (d, J = 1.8 Hz, 1H), 8.10 (s, 1H), 8.25 (d, J = 1.0 Hz, 1H), 10.31 (brs, 1H), 13.31 (brs, 1H).

TOF-MS (m/z); $418[M+1]^+$

Step 2

(E)-3-[2-(2-Benzoylaminophenyl)vinyl]-5-bromo-1H-indazole (1.38 g, 3.29 mmol) was suspended in tetrahydrofuran (70 mL), sodium hydride (476.4 mg, 11.91 mmol) was added thereto at room temperature in an argon atmosphere and the mixture was stirred at the same temperature for 10 minutes. Then, 12.5 mL (19.88 mmol) of n-butyl lithium (1.59 mol/L hexane solution) was added at -78 °C, the mixture was stirred at the same temperature for 25 minutes, N,N-dimethylformamide (5.1 mL, 66.08 mmol) was added thereto and temperature of the mixture was raised up to room temperature while stirring. Water was added to the reaction solution, the mixture was extracted with ethyl acetate, the organic layer was dried over anhydrous sodium sulfate and the solvent was evaporated in *vacuo.* The residue was purified by silica gel column chromatography (hexane/ethyl acetate = 2/1) to give (E)-3-[2-(2-benzoylaminophenyl)vinyl]-5-formyl-1H-indazole (724.2 mg, 60%).

$^1$H-NMR (270 MHz, DMSO-$d_6$)$\delta$; 7.34-7.43 (m, 3H), 7.53-7.68 (m, 5H), 7.73 (d, J = 16.7 Hz, 1H), 7.82 (d, J = 8.7 Hz, 1H), 8.02-8.05 (m, 1H), 8.08 (d, J = 1.5 Hz, 1H), 8.10 (s, 1H), 8.57 (s, 1H), 9.65 (s, 1H), 10.34 (s, 1H), 13.56 (brs, 1H).

TOF-MS (m/z); $368[M+1]^+$

Step 3

Toluene (70 mL) was added to a mixture of (E)-3-[2-(2-benzoylaminophenyl)vinyl]-5-formyl-1H-indazole (710.7 mg, 1.93 mmol) and methyl triphenylphosphoranylideneacetate (973.1 mg, 2.91 mmol) followed by heating to reflux for 2.5 hours. The resulting precipitate was filtered to give (E)-3-[2-(2-benzoylaminophenyl)vinyl]-(E)-5-(2-methoxycarbonylvinyl)-1H-indazole (781.4 mg, 85%).

$^1$H-NMR (270 MHz, DMSO-$d_6$)$\delta$; 3.77 (s, 3H), 6.64 (d, J = 15.8 Hz, 1H), 7.14-7.29 (m, 1H), 7.36-7.41 (m, 2H), 7.52-7.79 (m, 7H), 7.70 (d, J = 16.8 Hz, 1H), 7.97-8.01 (m, 1H), 8.07-8.10 (m, 2H), 8.35 (s, 1H), 10.32 (s, 1H), 13.33 (brs, 1H).

TOF-MS (m/z); $422[M-1]^-$

Step 4

(E)-3-[2-(2-benzoylaminophenyl)vinyl]-(E)-5-(2-methoxycarbonylvinyl)-1H-indazole (560.2 mg, 1.32 mmol) was dissolved in tetrahydrofuran (55 mL), diisobutyl aluminum hydride (1.0 ml/L toluene solution) (8.5 mL, 8.84 mmol) was added thereto at -78 °C, the mixture was stirred at the same temperature for 1 hour and temperature thereof was gradually raised up to room temperature followed by stirring for additional 1 hour. The reaction was stopped by addition of water, then aqueous solution of sodium potassium (+)-tartrate and ethyl acetate were added and the mixture was

stirred at room temperature for 1 hour. The aqueous layer was extracted with ethyl acetate, the organic layer was washed with saturated brine and dried over anhydrous sodium sulfate and the solvent was evaporated in *vacuo.* The residue was purified by silica gel column chromatography (chloroform/methanol = 50/1) to give Compound 25 (462.0 mg, 88%).

$^1$H-NMR (270 MHz, DMSO-$d_6$)δ; 4.02-4.10 (m, 2H), 4.78-4.88 (m, 1H), 6.26 (dt, J = 4.8 Hz, 15.8 Hz, 1H), 6.37 (d, J = 15.8 Hz, 1H), 7.34-7.37 (m, 3H), 7.45-7.66 (m, 6H), 7.60 (d, J = 16.2 Hz, 1H), 7.87 (s, 1H), 7.97-8.00 (m, 1H), 8.08 (d, J = 1.6 Hz, 1H), 8.11 (s, 1H), 10.31 (brs, 1H), 13.13 (brs, 1H).

TOF-MS (m/z); 394$[M-1]^-$

Example 24 (E)-3-[2-(2-Benezoylaminophenyl)vinyl]-5-(3-hydroxy-1-propyn-1-yl)-1H-indazole (Compound 26)

Step 1

Ethyl 5-iodo-1H-indazole-3-carboxylate (1.432 g, 4.53 mmol) prepared according to a known method *[Heterocycles,* volume 43, page 2701 (1996)] was treated with diisobutylaluminum hydride (1.0 mol/L toluene solution, 27 mL, 28.08 mmol) by a conventional method to give 3-hydroxymethyl-5-iodo-1H-indazole (883.2 mg, 71%).

$^1$H-NMR (270 MHz, DMSO-$d_6$)δ; 4.75 (d, J = 5.9 Hz, 2H), 5.27 (t, J = 5.9 Hz, 1H), 7.35 (d, J = 8.9 Hz, 1H), 7.56 (d, J = 8.9 Hz, 1H), 8.24 (s, 1H), 12.93 (brs, 1H).

TOF-MS (m/z); 272$[M-1]^-$

Step 2

3-Hydroxymethyl-5-iodo-1H-indazole (1.849 g, 6.75 mmol) and triphenylphosphonium bromide (2.31 g, 6.73 mmol) were heated under reflux for one night in acetonitrile (150 mL). After cooling the mixture down to room temperature, insoluble matter was filtered off and the filtrate was concentrated *in vacuo*. The residue was subjected to trituration with acetonitrile to give (5-iodo-1H-indazol-3-ylmethyl) triphenylphosphonium bromide (3.594 g, 89%).

$^1$H-NMR (270 MHz, DMSO-$d_6$)δ; 5.59 (d, J = 15.2 Hz, 2H), 7.30 (d, J = 8.9 Hz, 1H), 7.50 (d, J = 8.9 Hz, 1H), 7.66-7.85 (m, 16H), 13.32 (brs, 1H).

TOF-MS (m/z); 519$[M-79]^+$

Step 3

In a similar manner to Example 14, (E)-3-[2-(2-benzoylaminophenyl)vinyl]-5-iodo-1H-indazole (3.373 g, 76%) was prepared from (5-iodo-1H-indazol-3-ylmethyl)triphenylphosphonium bromide (5.753 g, 9.60 mmol), N-(2-formylphenyl)benzamide (2.161 g, 9.59 mmol) and potassium carbonate (3.977 g, 28.77 mmol).

$^1$H-NMR (270 MHz, DMSO-$d_6$)δ; 7.34-7.41 (m, 4H), 7.51-7.65 (m, 6H), 8.00 (dd, J = 5.4 Hz, 5.8 Hz, 1H), 8.07 (d, J = 1.8 Hz, 1H), 8.10 (d, J = 1.3 Hz, 1H), 8.24 (s, 1H), 10.31 (brs, 1H), 13.28 (brs, 1H).

TOF-MS (m/z); 466$[M+1]^+$

Step 4

Triethylamine (1.1 mL, 7.88 mmol) and then 3-tert-butyldimethylsilyloxy-1-propyne (0.121 mL, 0.60 mmol) were added to a mixture of (E)-3-[2-(2-benzoylaminophenyl)vinyl]-5-iodo-1H-indazole (139.2 mg, 0.30 mmol), dichlorobis(triphenylphosphine)palladium (32.6 mg, 0.046 mmol) and cuprous iodide (8.6 mg, 0.45 mmol) followed by stirring at 60 °C for 1 hour. After cooling to room temperature, water was added thereto and the mixture was extracted with ethyl acetate. The organic layer was washed with water and saturated brine successively, dried over anhydrous magnesium sulfate and the solvent was evaporated in *vacuo.* The residue was purified by silica gel column chromatography (hexane/ethyl acetate = 5/1) to give (E)-3-[2-(2-benzoylaminophenyl)vinyl]-5-(3-tert-butyldimethylsilyloxy-1-propyn-1-yl)-1H-indazole (62.2 mg, 41%).

$^1$H-NMR (270 MHz, $CDCl_3$)δ; 0.17 (s, 6H), 0.94 (s, 9H), 4.52 (s, 2H), 7.20-7.67 (m, 8H), 7.91-8.01 (m, 4H), 10. 33 (brs, 1H).

TOF-MS (m/z); 506$[M-1]^-$

Step 5

(E)-3-[2-(2-Benzoylaminophenyl)vinyl]-5-(3-tert-butyldimethylsilyloxy-1-propyn-1-yl)-1H-indazole (42.5 mg, 0.083 mmol) was dissolved in tetrahydrofuran (1 mL), tetrabutylammonium fluoride (1.0 ml/L tetrahydrofuran solution) was added thereto and the mixture was stirred at room temperature for 3 hours. Water was added to the reaction

solution, the mixture was extracted with ethyl acetate, the organic layer was washed with water and saturated brine successively and dried over anhydrous sodium sulfate and the solvent was evaporated *in vacuo*. The residue was purified by preparative thin-layer chromatography (chloroform/methanol = 9/1) to give Compound 26 (30.2 mg, 92%).

$^1$H-NMR (270 MHz, DMSO-$d_6$)δ; 4.31 (d, J = 5.7 Hz, 2H), 5.32 (t, J = 5.7 Hz, 1H), 7.34-7.42 (m, 4H), 7.51-7.66 (m, 6H), 7.99 (dd, J = 5.3 Hz, 5.6 Hz, 1H), 8.06 (d, J = 1.6 Hz, 1H), 8.09 (s, 1H), 8.15 (s, 1H), 10.29 (brs, 1H), 13.29 (brs, 1H).

TOF-MS (m/z); 392[M-1]$^-$

Reference Example 1 (E)-5-Nitro-3-[2-(3-pyridyl)-vinyl] -1H-indazole (Compound D)

Under cooling with ice, concentrated sulfuric acid (0.013 mL, 2.5 mmol) was added to concentrated nitric acid (0.013 mL, 2.9 mmol) and then Compound 2 (93 mg, 0.42 mmol) was added thereto followed by stirring at room temperature for 1 hour. The reaction solution was made alkaline with an aqueous solution of 10 mol/L sodium hydroxide, concentrated to dryness *in vacuo* and recrystallized from ethanol to give Compound D (51 mg, 45%).

$^1$H-NMR (270 MHz, DMSO-$d_6$)δ; 7.43 (dd, J = 4.7 Hz, 7.7 Hz, 1H), 7.62 (d, J = 16.6 Hz, 1H), 7.73 (d, J = 9.2 Hz, 1H), 7.90 (d, J = 16.6 Hz, 1H), 8.20-8.24 (m, 2H), 8.49 (dd, J = 1.3 Hz, 4.7 Hz, 1H), 8.93 (d, J = 1.3 Hz, 1H), 9.21 (d, J = 2.0 Hz, 1H), 13.86 (brs, 1H).

TOF-MS (m/z); 267[M+1]$^+$

Reference Example 2 (E)-5-Bromo-3-[2-(3-pyridyl)vinyl] -1H-indazole (Compound E)

Step 1

Ethyl 5-bromo-1H-indazole-3-carboxylate (16.44 g, 61.09 mmol) prepared according to a known method [*J. Am. Chem. Soc.*, volume 74, page 2009 (1952)] was dissolved in tetrahydrofuran (610 mL), a toluene solution of 1.0 mol/L diisobutyl aluminum hydride (305 mL, 305 mmol) was added over 20 minutes period under nitrogen stream at -78 °C and temperature thereof was gradually raised up to 0 °C while stirring, followed by further stirring for 3 hours. To the reaction solution was added sodium sulfate decahydrate (98.23 g, 304.9 mmol). This was stirred for one night, the mixture was dried over anhydrous sodium sulfate and filtered through Celite, and the filtrate was concentrated *in vacuo* to give 5-bromo-3-hydroxymethyl-1H-indazole (13.81 g, quantitatively).

Rf = 0.5 (chloroform/methanol = 9/1)

$^1$H-NMR (270 MHz, DMSO-$d_6$)δ; 5.04 (d, J = 5.9 Hz, 2H), 5.76 (t, J = 5.9 Hz, 1H), 7.67-7.74 (m, 2H), 8.30 (s, 1H).

Step 2

Acetonitrile (3.8 mL) and triphenylphosphonium bromide (301 mg, 0.877 mmol) were added to 5-bromo-3-hydroxymethyl-1H-indazole (100 mg, 0.440 mmol) followed by heating to reflux for 1 hour. This was cooled down to room temperature, insoluble matters were filtered off and the filtrate was concentrated *in vacuo.* The residue was subjected to trituration with a mixed solvent of acetonitrile-diethyl ether to give (5-bromo-1H-indazol-3-ylmethyl)triphenylphosphonium bromide (75 mg, 32%).

$^1$H-NMR (270 MHz, DMSO-$d_6$)δ; 5.58 (d, J = 15.2 Hz, 2H), 7.24-8.05 (m, 18H), 13.35 (brs, 1H).

Step 3

In a similar manner to Reference Example 5, the compound E (5.8 mg, 58%) was prepared from (5-bromo-1H-indazol-3-ylmethyl)triphenylphosphonium bromide (20 mg, 0.036 mol), pyridine-3-carboxaldehyde (0.0031 mL, 0.033 mmol) and potassium carbonate (15 mg, 0.11 mmol).

$^1$H-NMR (270 MHz, DMSO-$d_6$)δ; 7.35 (dd, J = 4.8 Hz, 7.7 Hz, 1H), 7.42-7.50 (m, 2H), 7.47 (d, J = 17.0 Hz, 1H), 7.64 (d, J = 17.0 Hz, 1H), 8.12 (d, J = 7.7 Hz, 1H), 8.40 (m, 2H), 8.80 (s, 1H), 13.35 (brs, 1H).

TOF-MS (m/z); 298[M-1]$^-$

Reference Example 3 (E)-5-Formyl-3-[2-(3-pyridyl)-vinyl]-1H-indazole (Compound F)

Compound E (2.03 g, 6.76 mmol) was dissolved in tetrahydrofuran (100 mL), sodium hydride (298 mg, 7.44

mmol) was added thereto while cooling with ice, the mixture was stirred for 40 minutes and then cooled down to -78 °C, n-butyl lithium (1.56 mol/L hexane solution, 6.5 mL, 10 mmol) was added thereto and the mixture was stirred at same temperature for 1 hour. To the reaction solution was added N,N-dimethylformamide (1.57 mL, 20.3 mmol) and the mixture was poured into a saturated aqueous solution of sodium bicarbonate while stirring at the same temperature followed by extracting with ethyl acetate. The organic layer was washed with a saturated brine and dried over anhydrous magnesium sulfate and the solvent was evaporated in *vacuo.* The residue was recrystallized from a mixed solvent of water/2-propanol to give Compound F (888 mg, 56%)

$^1$H-NMR (270 MHz, DMSO-$d_6$)δ; 7.44 (dd, J = 4.6 Hz, 7.9 Hz, 1H), 7.64 (d, J = 16.8 Hz, 1H), 7.69 (d, J = 8.9 Hz, 1H), 7.78 (d, J = 16.8 Hz, 1H), 7.88 (dd, J = 1.3 Hz, 8.9 Hz, 1H), 8.21 (dt, J = 2.0 Hz, 7.9 Hz, 1H), 8.49 (dd, J = 2.0 Hz, 4.6 Hz, 1H), 8.88 (s, 1H), 8.91 (d, J = 2.0 Hz, 1H), 10.08 (s, 1H).

TOF-MS (m/z); 250$[M+1]^+$

Reference Example 4 (E)-5-Amino-3-[2-(3-pyridyl)vinyl] -1H-indazole (Compound G)

Compound D (1.02 g, 3.84 mmol) was suspended in ethanol (200 mL), 10% palladium-carbon (50% aqueous product, 512 mg) was added thereto, then hydrazine monohydrate (1.87 mL, 38.4 mmol) was added thereto while stirring at 70 °C and the mixture was further stirred for 40 minutes at the same temperature. The reaction mixture was quickly cooled down to 0 °C, filtered through Celite and the filtrate was concentrated *in vacuo*. The residue was purified by silica gel column chromatography (chloroform/methanol = 15/1) and subjected to trituration with chloroform to give Compound G (592 mg, 65%).

$^1$H-NMR (270 MHz, DMSO-$d_6$)δ; 4.89 (brs, 2H), 6.82 (dd, J = 2.0 Hz, 8.9 Hz, 1H), 7.17 (brs, 1H), 7.26 (d, J = 8.9 Hz, 1H), 7.27 (d, J = 16.7 Hz, 1H), 7.39 (dd, J = 4.6 Hz, 8.1 Hz, 1H), 7.55 (d, J = 16.7 Hz, 1H), 8.09 (d, J = 8.1 Hz, 1H), 8.43 (dd, J = 1.8 Hz, 4.6 Hz, 1H), 8.78 (d, J = 1.8 Hz, 1H), 12.80 (brs, 1H).

TOF-MS (m/z); 237$[M+1]^+$

Reference Example 5 (E)-3-[2-(3-Pyridyl)vinyl]-1H-indazole (Compound 2)

(1H-Indazol-3-ylmethyl)triphenylphosphonium iodide (10.0 g, 19.2 mmol) was suspended in methanol (120 mL), 3-pyridinecarboxaldehyde (1.8 mL, 19 mmol) and potassium carbonate (7.96g, 57.6 mmol) were added thereto and the mixture was stirred at room temperature for 1. 5 hours. Water was added to the reaction solution, the mixture was extracted with ethyl acetate, the organic layer was extracted with 1 mol/L hydrochloric acid, the aqueous layer was made alkaline with a 10 mol/L aqueous solution of sodium hydroxide and the resulting precipitate was filtered and recrystallized with a mixed solvent of ethanol-water to give Compound 2 (2.14g, 50%).

$^1$H-NMR (270 MHz, DMSO-$d_6$)δ; 7.21 (dd, J = 7.3 Hz, 8.3 Hz, 1H), 7.40 (dd, J = 7.3 Hz, 8.6 Hz, 1H), 7.41 (m, 1H), 7.51 (d, J = 16.8 Hz, 1H), 7.55 (d, J = 8.6 Hz, 1H), 7.67 (d, J = 16.8 Hz, 1H), 8.16 (d, J = 10.2 Hz, 1H), 8.19 (d, J = 8.3 Hz, 1H), 8.45 (d, J = 4.6 Hz, 1H), 8.55 (s, 1H).

TOF-MS (m/z); 222$[M+1]^+$

Formulation Example 1 (Tablets)

A tablet having the following formulation is prepared in a conventional manner.

| | |
|---|---|
| Compound 2 | 5 mg |
| Lactose | 60 mg |
| Potato starch | 30 mg |
| Poly(vinyl alcohol) | 2 mg |
| Magnesium stearate | 1 mg |
| Tar dye | trace |

Industrial Applicability

According to the present invention, JNK inhibitors useful for treatment of a brain neurodegenerative disorder or the like, or novel indazole derivatives or pharmaceutically acceptable salts thereof which have JNK inhibitory activity and are useful for treatment of a brain neurodegenerative disorder or the like, are provided.

## Claims

1. A JNK (c-Jun N-terminal kinase) inhibitor comprising, as an active ingredient, an indazole derivative represented by Formula (I)

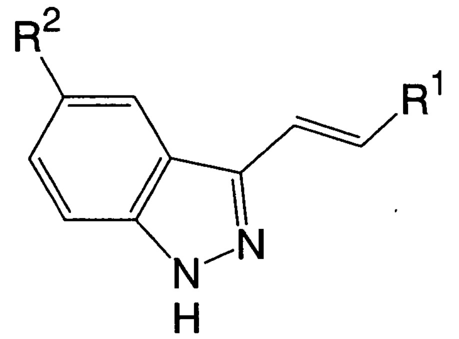

(I)

[wherein $R^1$ represents substituted or unsubstituted aryl or a substituted or unsubstituted heterocyclic group, and

$R^2$ represents

a) a hydrogen atom,
b) $NR^3R^4$ (wherein $R^3$ and $R^4$ may be the same or different and each represents a hydrogen atom, substituted or unsubstituted lower alkanoyl, substituted or unsubstituted aroyl, heteroaroyl, or lower alkoxycarbonyl),
c) carboxy,
d) $CONR^{3A}R^{3B}$ (wherein $R^{3A}$ and $R^{4B}$ may be the same or different and each represents a hydrogen atom, substituted or unsubstituted lower alkanoyl, substituted or unsubstituted aryl, substituted or unsubstituted aroyl, heteroaroyl or lower alkoxycarbonyl),
e) substituted or unsubstituted lower alkenyl or
f) substituted or unsubstituted lower alkynyl] or a pharmaceutically acceptable salt thereof.

2. An indazole derivative represented by Formula (II)

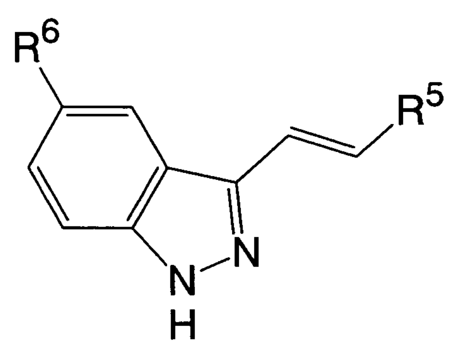

(II)

[wherein $R^5$ represents substituted or unsubstituted pyridyl, or aroylamino-substituted phenyl, and
$R^6$ represents

a) $NR^{3B}R^{4B}$ (wherein $R^{3B}$ and $R^{4B}$ may be the same or different and each represents a hydrogen atom, substituted or unsubstituted alkanoyl, substituted or unsubstituted aroyl or heteroaroyl),
b) carboxy,
c) $CONR^{3C}R^{3C}$ (wherein $R^{3C}$ and $R^{4C}$ may be the same or different and each represents a hydrogen atom or

substituted or unsubstituted aryl),
d) substituted or unsubstituted lower alkenyl or
e) substituted or unsubstituted lower alkynyl] or a pharmaceutically acceptable salt thereof.

**3.** A therapeutic agent for a brain neurodegenerative disorder comprising at least one indazole derivative or pharmaceutically acceptable salt thereof described in Claim 1.

**4.** The therapeutic agent for a brain neurodegenerative disorder according to Claim 3, wherein the brain neurodegenerative disorder is the disease selected from cerebral infarction, Parkinson's disease, Alzheimer's disease, progressive supranuclear paralysis, AIDS encephalopathy, transmissible spongiform encephalopathy, multiple sclerosis, amyotrophic lateral sclerosis, multiple system atrophy, attention-deficit/hyperactivity disorder, Huntington's disease, a diabetic neurosis and a traumatic neurodegenerative disorder.

**5.** A therapeutic agent for acute cerebral infarction comprising at least one indazole derivative or pharmaceutically acceptable salt thereof described in Claim 1.

**6.** A therapeutic agent for a brain neurodegenerative disorder comprising at least one indazole derivative or pharmaceutically acceptable salt thereof described in Claim 2.

**7.** The therapeutic agent for a brain neurodegenerative disorder according to Claim 6, wherein the brain neurodegenerative disorder is the disease selected from cerebral infarction, Parkinson's disease, Alzheimer's disease, progressive supranuclear paralysis, AIDS encephalopathy, transmissible spongiform encephalopathy, multiple sclerosis, amyotrophic lateral sclerosis, multiple system atrophy, attention-deficit/hyperactivity disorder, Huntington's disease, a diabetic neurosis and a traumatic neurodegenerative disorder.

**8.** A therapeutic agent for acute cerebral infarction comprising at least one indazole derivative or pharmaceutically acceptable salt thereof described in Claim 2.

**9.** A pharmaceutical composition comprising at least one indazole derivative or pharmaceutcally acceptable salt thereof described in Claim 2.

**10.** A JNK inhibitor comprising the indazole derivative or the pharmaceutically acceptable salt thereof described in Claim 2.

**11.** A method for treatment and/or prevention of a disease derived from activation of JNK, comprising a step of administering an effective amount of the indazole derivative or the pharmacologically acceptable salt thereof described in Claim 1.

**12.** A method for treatment and/or prevention of a brain neurodegenerative disorder, comprising a step of administering an effective amount of the indazole derivative or the pharmaceutically acceptable salt thereof described in Claim 1.

**13.** A method for treatment and/or prevention of acute cerebral infarction, comprising a step of administering an effective amount of the indazole derivative or the pharmaceutically acceptable salt thereof described in Claim 1.

**14.** A method for treatment and/or prevention of a disease derived from activation of JNK, comprising a step of administering an effective amount of the indazole derivative or the pharmaceutically acceptable salt thereof described in Claim 2.

**15.** A method for treatment and/or prevention of a brain neurodegenerative disorder, comprising a step of administering an effective amount of the indazole derivative or the pharmaceutically acceptable salt thereof described in Claim 2.

**16.** A method for treatment and/or prevention of acute cerebral infarction, comprising a step of administering an effective amount of the indazole derivative or the pharmaceutically acceptable salt thereof described in Claim 2.

**17.** Use of the indazole derivative or the pharmaceutically acceptable salt thereof described in Claim 1 for the manufacture of a JNK inhibitor.

**18.** Use of the indazole derivative or the pharmaceutically acceptable salt thereof described in Claim 1 for the manufacture of an agent for treatment and/or prevention of a brain neurodegenerative disorder.

**19.** Use of the indazole derivative or the pharmaceutically acceptable salt thereof described in Claim 1 for the manufacture of an agent for treatment and/or prevention of acute cerebral infarction.

**20.** Use of the indazole derivative or the pharmaceutically acceptable salt thereof described in Claim 2 for the manufacture of a JNK inhibitor.

**21.** Use of the indazole derivative or the pharmaceutically acceptable salt thereof described in Claim 2 for the manufacture of an agent for treatment and/or prevention of a brain neurodegenerative disorder.

**22.** Use of the indazole derivative or the pharmaceutically acceptable salt thereof described in Claim 2 for the manufacture of an agent for treatment and/or prevention of acute cerebral infarction.

## INTERNATIONAL SEARCH REPORT

International application No. PCT/JP03/15481

**A. CLASSIFICATION OF SUBJECT MATTER**

Int.Cl[7] A61K31/416, 31/4439, 31/444, 31/497, A61P3/10, 9/00, 9/10, 21/00, 25/00, 25/14, 25/16, 25/28, 43/00, C07D231/56, 401/06, 401/14, 409/14, 403/12

According to International Patent Classification (IPC) or to both national classification and IPC

**B. FIELDS SEARCHED**

Minimum documentation searched (classification system followed by classification symbols)

Int.Cl[7] A61K31/416, 31/4439, 31/444, 31/497, A61P3/10, 9/00, 9/10, 21/00, 25/00, 25/14, 25/16, 25/28, 43/00, C07D231/56, 401/06, 401/14, 409/14, 403/12

Documentation searched other than minimum documentation to the extent that such documents are included in the fields searched

Electronic data base consulted during the international search (name of data base and, where practicable, search terms used)

STN/CAS, JOIS

**C. DOCUMENTS CONSIDERED TO BE RELEVANT**

| Category* | Citation of document, with indication, where appropriate, of the relevant passages | Relevant to claim No. |
|---|---|---|
| X<br><br>Y | WO 02/10137 A2 (SIGNAL PHARMACEUTICALS, INC.), 07 February, 2002 (07.02.02), & US 2002/103229 A1 & EP 1313711 A2 | 1-4,6-10, 17-18,20-22<br>5,19 |
| Y | YANG, D.D. et al., "Absence of excitotoxicity-induced apoptosis in the hippocampus of mice lacking the Jnk3 gene", Nature, Vol.389, pages 865 to 870, (1997) | 5,19 |
| X<br>A | WO 01/53268 A2 (AGOURON PHARMACEUTICALS, INC.), 26 July, 2001 (26.07.01), & EP 1250326 A2 & US 2002/161022 A1 & US 6555539 B2 & BR 2001007783 A & JP 2003-520273 A & EE 200200398 A & NO 2002002117 A & BR 107011 A & US 2003/139463 A1 | 2,9<br>1,3-8,17-22 |

[X] Further documents are listed in the continuation of Box C. [ ] See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| | |
|---|---|
| Date of the actual completion of the international search<br>10 February, 2004 (10.02.04) | Date of mailing of the international search report<br>24 February, 2004 (24.02.04) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/15481

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2-32059 A (Kyowa Hakko Kogyo Co., Ltd.), 01 February, 1990 (01.02.90), (Family: none) | 2,9<br>1,3-8,17-22 |
| P,X | WO 03/101968 A1 (Eisai Co., Ltd.), 11 December, 2003 (11.12.03), (Family: none) | 1-4,6-10, 17-18,20-22 |
| A | WO 2001/02369 A2 (AGOURON PHARMACEUTICALS, INC.), 11 January, 2001 (11.01.01),<br>& BR 2000012352 A & EP 1218348 A2<br>& JP 2003-503481 A & NZ 516676 A<br>& US 6531491 B1 & US 6534524 B1<br>& NO 2001005797 A & ZA 2001010061 A<br>& BR 106380 A | 1-10,17-22 |

Form PCT/ISA/210 (continuation of second sheet) (July 1998)

**INTERNATIONAL SEARCH REPORT**

International application No.
PCT/JP03/15481

**Box I Observations where certain claims were found unsearchable (Continuation of item 2 of first sheet)**

This international search report has not been established in respect of certain claims under Article 17(2)(a) for the following reasons:

1. [x] Claims Nos.: 11 to 16
because they relate to subject matter not required to be searched by this Authority, namely:
The inventions as set forth in claims 11 to 16 are relevant to method for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)